# EUROPEAN PATENT SPECIFICATION

(11) **EP 4 135 815 B1**
(45) Date of publication and mention of the grant of the patent: **09.07.2025**
(21) Application number: 21789023.5
(22) Date of filing: 08.04.2021
(51) Int. Cl.: A61M 16/20, A61M 16/10, A61M 16/00, A61M 16/08

(54) **HIGH-PERFORMANCE, LOW COST MEDICAL BREATHING GAS DELIVERY SYSTEMS**
HOCHLEISTUNGS- UND KOSTENGÜNSTIGE MEDIZINISCHE ATEMGASABGABESYSTEME
SYSTÈMES DE DISTRIBUTION DE GAZ RESPIRATOIRE MÉDICAL, TRÈS PERFORMANTS ET DE COÛT MODÉRÉ

(30) Priority: 15.04.2020 US 202063010207 P; 04.05.2020 US 202063019601 P
(43) Date of publication of application: 22.02.2023
(73) Proprietor: Horwitz, Christopher, Max, Pittsburgh, PA 15217 (US)
(72) Inventor: Horwitz, Christopher, Max, Pittsburgh, PA 15217 (US)
(74) Representative: Haseltine Lake Kempner LLP
(86) International application number: PCT/US2021/026368
(87) International publication number: WO 2021/211350

(56) References cited:
- EP-B1- 0 960 629
- EP-B1- 0 960 630
- WO-A1-2016/057686
- US-A- 4 278 110
- US-A- 5 299 568
- US-A1- 2007 221 221
- US-A1- 2011 041 848
- US-A1- 2016 252 912
- US-A1- 2016 252 912
- US-A1- 2016 287 824
- US-A1- 2019 167 928

## Description

### BACKGROUND

The following information is provided to assist the reader in understanding technologies disclosed below and the environment in which such technologies may typically be used. The terms used herein are not intended to be limited to any particular narrow interpretation unless clearly stated otherwise in this document. References set forth herein may facilitate understanding of the technologies or the background thereof.

While the discussion below relates mainly to patients experiencing respiratory distress, the discussion of equipment characteristics, requirements, and improvements herein applies equally to possible uses in anesthesia and in care of paralytic patients.

Representative embodiments of devices, systems and methods hereof introduce novel and simple medical ventilator designs which can be used for breathing assistance in patients with reduced lung function. Such ventilators have been in common use since the early 1900's. The polio epidemic saw widespread use of iron lungs (for example, US Patent No. 2,240,838); later implementations (for example, US Patent Nos. 2,473,416 and 3,347,228) used smaller devices feeding air and oxygen under pressure into tubes inserted into the nose, mouth, or trachea, and sealed against the inner tracheal wall ("intubation").

Ventilators both cycle, and extensively monitor, pressures and flows to cause, or assist, breathing action and to introduce added gases such as oxygen and anesthetics without causing lung damage. Various operating modes and signal waveforms have been employed with 'pressure control', 'volume control', 'time controlled adaptive ventilation' 'PEEP' (Positive End-Expiratory Pressure) and combinations thereof. See, for example, G. F. Nieman et al, "Prevention and treatment of acute lung injury with time-controlled adaptive ventilation: ...", Ann Intensive Care (2020) 10:3 (DOI.org/10.1185/s13613-019-0619-3). Additionally, lowered mortality rates in patients ventilated with high-frequency pressure cycling demonstrate the need for ventilators that can provide not only slow pressure cycling, but also complex flow waveforms that hold lungs open yet cycle oxygen and carbon dioxide gases effectively. See, for example, Krishnan JA, Brower RG, "High-frequency ventilation for acute lung injury and ARDS" Chest 118 (3): 795-807 doi:10.1378/chest.118.3.795 PMID 10988205 (2000); and Rimensberger PC "ICU cornerstone: high-frequency ventilation is here to stay" Critical Care 7 (5): 342-4 doi:10.1186/cc2327 PMC 270713 PMID 12974963 (October 2003).

Ventilators often also provide PEEP (Positive End-Expiratory Pressure) to help avoid airway and lung collapse after the patient breathes out. There is similar widespread domestic use of CPAP devices from the early 2000's, providing Constant Positive Airway Pressure to help keep airways open and prevent sleep apnea, using masks to deliver an elevated air pressure to the nose and mouth. See, for example, PCT Publication No. WO 2008/108789A1.

Ventilators may become scarce during potential pandemics, which have occurred roughly every 4 years since 2003. See, for example, S. Perlman, "Another Decade, Another Coronavirus" New England J. Med 2020; 382:760-762 DOI: 10.1056/NEJMe2001126 (2020). If permitted to attain an infection doubling time of 2-3 days, communities risk excess mortality rates due to limited supplies of ventilators, hospital beds, and providers. Such a pandemic, COVID-19, was permitted to become widespread by many countries in 2020, with grim results. L. Rosenbaum "Facing Covid-19 in Italy - Ethics, Logistics, and Therapeutics on the Epidemic's Front Line" New England J. Med 2020; DOI: 10.1056/NEJMp2005492 (2020). Developing-world countries are even more hard-hit, with similar infection rates and more limited resources.

Pandemics which cause Acute Respiratory Distress Syndrome (ARDS), such as the 2019 COVID-19 pandemic, experience mortality rates above 39% in intubated patients (Nieman, ibid) even using modern ventilators, since ARDS occurs when a large lung fraction is inflamed and in a congested, delicate state. See, for example, N. Zhu et al"A Novel Coronavirus from Patients with Pneumonia in China, 2020" New England J. Med 2020; 382:727-733 DOI: 10.1056/NEJMoa2001017 (2020). Such congested lungs do not readily oxygenate, yet are easily damaged by the pressures that may be needed for oxygenation (Nieman, ibid). In addition, some are finding that the normal ventilator ARDS protocol may be completely inappropriate and may kill patients at a 60% rate when COVID-19 patients present without lung congestion, rendering accurate and dynamic adjustment to patient status even more urgent. L. Gattinoni et al, "COVID-19 pneumonia: different respiratory treatment for different phenotypes?" Intensive Care Medicine; DOI: 10.1007/s00134-020-06033-2 (2020). Ventilator manufacturers in such pandemics experience demand which they are unable to accommodate, with custom component and workforce availability being important limits. To help meet the need, CPAP devices can assist in early and later stages of ARDS, but ventilators are still required during the acute phase of infection.

Mechanical ventilators from the 1940's onward used devices such as moving pistons, bellows, and diaphragms to move gases in and out of patient lungs, with trigger mechanisms to provide cyclic action. Later models incorporated additional triggering pneumatics and electronics to respond to patient demand, as detected in airway pressure fluctuations, thus guiding the ventilation cycle rate. See, for example, US Patent 5,107,830 and US Patent 3,379,194. More recent developments use computer control and more compact designs, some of which can also be used in the home with face masks. See, for example, US Patent Nos. 10,286,169, U.S. Patent Publication Nos. 2019/0054268 and 2003/0230307 A1, and EP0960630B1.

However even such more modem designs use a quantity of valves, pressure regulators, precisely calibrated adjustable flow control valves, and other specially sourced components. For example, a number of currently available ventilator designs may use 6 valves, a pump-action line, and 2 pressure sensors: 10 valves, 1 oxygen sensor, 1 high-pressure fan, and 5 pressure sensors; 1 high-pressure fan, 13 valves, and 4 pressure sensors.

All of the above require dedicated special component sourcing, and complex tubing and assembly. Some of those valves guarantee the necessary safe operation by permitting free breathing in case of power or pressure loss, and also by venting overpressures in case of control failure; the others make complex operations possible, but with complexity costs.

Anesthesia machines are typically simpler than ventilators, often being manually controlled with fluidic actuators and feedback rather than by electronics and electric valves, but can suffer from regulator unreliability due to the small sensing areas available for the low airway pressures, with the attendant possibility of danger to patients during operations if there are poorly-controlled airway pressure bursts and dumps.

There is thus a need for advanced ventilator designs, and other systems for delivering breathing gas to a patient's airways, capable of delivering precisely metered gases, guaranteeing patient safety in case of equipment malfunction, and monitoring patient breathing status and history, but which use readily available standard components which are easily assembled. In addition, precise blending of oxygen into the air stream would enable operation without requiring feedback control from oxygen sensors, which operate at high temperature and have their own accuracy and reliability concerns. Precise blending of other gases would additionally make such improved ventilators suitable for operating room anesthesia machines.

Embodiments disclosed in this document meet those goals for construction of an advanced ventilator, requiring at minimum an electronic control such as manually set level controls, or a computer control, with one or two easily obtained valves, a simple manifold construction, and two pressure sensors, to attain controlled and calibrated air feed. Adding one additional valve and pressure sensor for each additional gas in the mixture permits precise narcotic blends, and oxygen blends without the need for oxygen sensor feedback control.

### SUMMARY

In one aspect, a method of air supply to patient airways includes flowing a controlled flow of gas through a one-way valve in a gas distribution manifold into an interstitial space fluidly connected to ports attached to the patient breathing tube, a patient airway pressure sensor or gauge, a second one-way valve permitting patient air intake draw in case of system failure, and an exhalation valve connected controllably to a proportioning valve drive such that the interstitial space's operating and exhalation pressures can be controlled dynamically, while also ensuring that the valve will permit exhaled gases to escape in case of system failure (that is, mechanical failure and/or power loss).

An additional pressure relief valve may be installed to permit interstitial space overpressure to be vented, independent of mechanical or electronic failure, or of maladjustment, of the exhalation valve.

A second patient airway pressure sensor or gauge connection may be placed at a predetermined point along the patient airway tube line or after a restricting orifice at the patient port outlet of the manifold in order to directly monitor patient inhaled and exhaled air flow from the differential pressure signal between the patient and the interstitial space of the manifold, and to use that thus separated and buffered patient volume for more accurately determining patient pressure demand signals.

The interstitial space may be divided into a first (inhalation) interstitial space and a second (exhalation) interstitial space in cases where two patient ventilating tubes (an inhalation tube and an exhalation tube) are desired; for inhalation flow direct from the input gas supplies; and for exhalation directly to an exhale valve line. An overpressure valve may be placed either directly ported to the patient intubation connection or to the second (exhalation) interstitial space. One-way check valves in such a case may be installed on the block manifold described herein in fluid connection with the inhalation tube and the exhalation tube, respectively, or may be pre-installed in the patient inhalation and exhalation tubes close to the patient intubation connection.

The drive mechanism for delivery of pressurized gas into the manifold may be a high-pressure variable-speed fan with a known [pressure - flow - rotation speed] characteristic, as is common in the industry, with varied fan speed providing the pressure required at any time to attain the desired flow, and with the interstitial space pressure further controlled by the exhale valve such that excess flow from the fan is vented.

Additionally, pressurized gas may be delivered from one or more compressed gas sources/tanks in fluid connection with the manifold. Such compressed gases may be fed into one or more gas flow controllers ("GFCs") configured, for example, according to US Patent No. 9,910,448. Each one of such gas flow controllers takes a feed of compressed gas into an upstream valve which is fluidly attached to a known calibration chamber volume at a known temperature capable of sufficiently averaging short-term flow variations as the upstream valve cycles between predetermined states, and a pressure gauge. The calibration chamber volume is then fluidly connected to the manifold through a restrictor device which permits the average flow to the manifold to be determined by control of the average pressure in the chamber volume relative to the manifold interstitial space pressure.

The restrictor device may, for example, be a thin orifice with a hole in it and with an effective thickness no greater than 4 times the orifice diameter, or a tube with an inner diameter smaller than 4 times the length of the tube. These restrictor types have differing characteristics; restrictor choice is determined by the flow range, available geometry, and transient performance required.

Additionally, the system can be used to dynamically measure and calibrate the absolute gas flow rate from the known chamber volume and temperature into the manifold by monitoring the calibration chamber pressure variations in real time as the valve cycles between predetermined states.

One of the (breathing) gas feed sources may be air. An additional (breathing) gas feed source may be oxygen. A further (breathing) gas feed source, if used for anesthesia, may be nitrous oxide. One or more other (breathing) gas compositions may, for example, be delivered for therapeutic and/or diagnostic purposes. Any such additional gas supply fed through a gas flow controller as described will provide a calibrated flow rate and accurate gas mixing without any additional sensor or feedback mechanism being required. As used herein, the term "breathing gas" refers to any gas to be delivered to a patient for respiration.

In any of these embodiments, the properties of the restrictors in the various feed systems may be stored in a data file for later access, permitting rapid ramp rates of flow levels to desired values by causing the calibrated volumes to attain the desired average pressure, hence to deliver the desired flow through their associated restrictor, without waiting for the auto-calibration process to verify restrictor calibration. In addition, historical changes in those stored restrictor properties would signal a maintenance requirement due to possible fouling or geometric changes, permitting the system to not only auto-calibrate, but also warn of required preventive maintenance before any loss of calibration accuracy may cause the system to malfunction.

A further valve may be applied to the above calibrated chamber volume to vent accumulated chamber pressure, thus rapidly terminating the flow of that gas, if the required rapid response cannot be accommodated by the manifold exhaust valve response function.

In the case of dynamic auto-calibration of gas flows according to US Patent No. 9,910,448 (C. Horwitz) any calibration chamber pressure sensor zero drift is cancelled out since only pressure differences are used to calibrate flows. Additionally, at low flow rates which require low calibration chamber pressures, any pressure sensor zero drift is able to be calculated given the known flow rate and restrictor properties, and applied to the pressure sensor readout such that rapid changes of flow, even at low flow and pressure levels, are auto-corrected for zero drift. This enables economical, readily available pressure sensors to be used with full system accuracy maintained.

In low-resource settings a computer control may not be feasible. In that case, manual adjustment of the gas flow controller valve duty cycle, combined with power control of the compressed gas source fan if this is present, and with readout of pressures either electronically or with dial gauges, can yield a serviceable controlled source of gas to the patient. An added oscillator providing a controllable duty cycle waveform would then cycle the exhale control valve between preset states, as well as the gas flow and fan controls, to yield a predetermined inhale/exhale waveform. Even such a simple system is capable of the auto-calibration of gas flow rates, if readout of either gas pressure rate-of-fall or maximum and minimum pressures during each pulse is presented to the operator.

If available, computer control would maintain pressure and flow waveforms without need for the above manual duty cycle adjustments, would enable a variety of operating modes, and would permit patient demand triggering of exhale and inhale cycles if desired. Such a computer control may also include network interfacing for data transmission, and data storage and data collection to enable graphs of cyclic waveforms to be presented. Such graphs may include waveforms of pressure, flow, and derived quantities such as volume of gas moved in various portions of the waveform, rates of change of pressure or flow with time in portions of the waveforms to enable lung health to be determined in a dynamic fashion, historical data collected and compared over time periods varying from, for example, minutes to weeks, and appropriate alarms if waveform characteristics move into dangerous regimes.

Such GFC systems as described herein use time-varying gas flow to provide calibrated average flow levels. The level of flow variation may be small relative to the average flow, or large; GFC calibrated volume pressure variations ranging between 0.1% and 100% of average pressure may be successfully employed in various embodiments hereof. In the case of small relative pressure variations, flow to the patient free of noticeable pulsations is attained. However, in the case of larger relative pressure variations, patient flow is modulated with a pulsatile waveform from the GFC, which is attenuated in amplitude by averaging in patient tubulation and lung volumes. Such pulsatile amplitudes can be adjusted in embodiments hereof with changes to patient tubulation geometry, manifold geometry, GFC geometry, and GFC valve drive, affording an additional clinical tool which may assist in severely congested ARDS patient care.

It has been shown that breakup of the viscous fluid in lung spaces is vital to recovery of lung function (Nieman, op cit). In more basic studies of fluid dynamics it has been shown that vibrations greatly enhance the breakup of fluid threads, by encouraging coalescence into droplets under conditions related to fluid viscosity and vibration frequency (eg, Javadi et al, "Delayed Capillary Breakup of Falling Viscous Jets, Phys. Rev. Lett. 110 (14), 144501 (2013), DOI: 10.1103 / PhysRevLett. 110.144501). Many clinical studies have likewise demonstrated benefits of pulsatile ventilator flow. See, for example, Krishnan and Brower, op cit; and Rimensberger, op cit. Thus, embodiments described herein yield clinical benefits not available from many current state-of-the-art ventilation systems.

In another aspect, a system to supply breathing gas to patient airways includes a gas distribution manifold, at least one controllable source of a breathing gas in fluid connection with the gas distribution manifold and including a pressurized tank of the breathing gas and a gas flow controller in fluid connection with an outlet of the pressurized tank and with the manifold, and a control system in operative connection with the gas flow controller and the gas distribution manifold. In a number of embodiments, the gas distribution manifold includes an interstitial space which may, for example, be divided into a first or inhalation space and a second or exhalation space to which various ports are in fluid connection. The gas flow controller may, for example, be in fluid connection with an outlet of the pressurized tank and with the gas inlet port. The control system may, for example, be in operative connection with the gas flow controller and with the gas distribution manifold. In other aspects, a method of implementing such a system to provide breathing gas to a patient and a method of manufacture of such as system are provided.

In still another aspect, gas distribution manifold for use in a system to deliver breathing gas to a patients airways, comprising: an interstitial space, at least one patient port in fluid connection with the interstitial space which is configured to connect to a patient breathing tube, at least a first pressure sensor in fluid connection with the interstitial space, an exhalation valve in fluid connection with the interstitial space, a controllable proportioning exhalation valve drive operatively connected to the exhalation valve to dynamically control pressures within the interstitial space, wherein the exhalation valve drive allows opening of the exhalation valve upon patient exhalation in case of system or power failure, an ambient air inlet port in fluid connection with the interstitial space and comprising a one-way valve to enable patient inhalation in case of system or power failure, and a gas inlet port in fluid connection with the interstitial space via a one-way valve to enable patient inhalation from that gas inlet port, wherein the gas distribution manifold is formed via sealed connection of a plurality of manifold blocks.. In a number of embodiments, the interstitial space may, for example, be divided into a first or inhalation space and a second or exhalation space to which the various ports are in fluid connection. In other aspects, a method of implementing such a gas distribution manifold to provide breathing gas to a patient and a method of manufacture of such as gas distribution manifold are provided.

An object hereof is to provide a system for patient ventilation that is highly accurate and which auto-calibrates flows during normal operation.

Another object hereof is to provide a ventilator comprising a manifold and at least one controlled gas source to enable all currently required ventilator functions.

Another object hereof is to provide a ventilator manifold which can be simply and quickly constructed to yield all of the required gas flow input and output connections with their associated valving.

Another object hereof is a ventilator manifold which can be constructed from readily available components.

Another object hereof is a manifold with enhanced reliability and serviceability with no internal fittings or tubing connections.

Another object hereof is to provide a system for patient ventilation that has no calibrated gas flow control or mixing valves.

Another object hereof is to provide a system for patient ventilation that does not require an oxygen sensor to attain high accuracy in oxygen mixture ratios.

Another object hereof is to provide a system for patient ventilation that provides accurate flow mixing on a real-time basis without requiring sensor feedback or calibrated flow control valves or calibrated flow restrictors.

Another object hereof is to provide a system for patient ventilation that is highly adaptable to various ventilation modes yet with a minimal hardware component requirement.

Another object hereof is to provide a system for patient ventilation that provides early warning of maintenance requirements, without losing accuracy in the meantime.

Another object hereof is to provide a system for patient ventilation with gas flow rates that are independent of pressure signal zero drift.

Another object hereof is to provide a system for patient ventilation that is compact and easily transported.

Another object hereof is to provide a system for patient ventilation that is able to be manufactured in low-resource settings.

Another object hereof is to provide a system for patient ventilation that is able to be manufactured in bulk, at high speed, and at low cost.

Another object hereof is to provide a system for patient ventilation that is highly reliable and easily serviced.

One embodiment of this technology comprises a single manifold with one pressure sensor, two flap valves for gas entry, and one exhalation valve, with air input provided at sufficient pressure for patient ventilation; and with control provided by simple electronic controls using manual adjustments.

Another embodiment of this technology comprises a single manifold with one pressure sensor, two flap valves for gas entry, and one exhalation valve, with air input provided at sufficient pressure for patient ventilation; with overall control, data storage, and display provided by a computer.

Another embodiment of this technology comprises one of the above assemblies with a patient flow restriction, which may be simply the tube leading from manifold to patient, and with a pressure sensor connected fluidly at the patient side of the restriction.

Another embodiment of this technology comprises any of the above embodiments with an additional one-way valve permitting excess pressures to be vented from the manifold interstitial space which connects fluidly with the patient line, supplementing the exhaust valve's venting action for additional patient protection.

Another embodiment of this technology comprises any of the above embodiments with a provision for more than one patient connection, typically for an inhalation line and an exhalation line, such that there are two interstitial spaces in the manifold for inhalation and exhalation, and with at least one pressure sensor monitoring the exhalation interstitial space pressure, and optionally with a second pressure sensor monitoring the inhalation interstitial space pressure.

Another embodiment of this technology comprises any of the above embodiments wherein at least one of the gas source pressures is provided by a fan.

Another embodiment of this technology comprises any of the embodiments above with at least one gas source pressure provided by a compressed gas source, fed first through a cyclically controlled valve into a calibration chamber monitored by a pressure sensor, and then through a restriction to the manifold's interstitial patient gas supply space. Said cyclically controlled valve may be a pressure regulator, a solenoid valve controlled by a pressure switch, a solenoid valve controlled by an oscillator, or a solenoid valve controlled by a computer controller.

Another embodiment of this technology comprises any of the above embodiments wherein one of the gases fed to the manifold is oxygen.

The present devices, systems, and methods, along with the attributes and attendant advantages thereof, will best be appreciated and understood in view of the following detailed description taken in conjunction with the accompanying drawings.

The present invention is defined by the appended claims.

### BRIEF DESCRIPTION OF THE DRAWINGS

Figure 1A shows a schematic embodiment of a system hereof with a manifold fed from both a fan, and from a "GFC" gas flow control containing a control valve-calibration volume-pressure sensor-restrictor combination.
Figure 1B shows a detailed manifold schematic in the case of a single patient breathing tube connection.
Figure 1C shows a detailed manifold schematic in the case of dual patient breathing tube connections.
Figure 2 shows a view of a manifold with a fan attached, for a single patient breathing tube connection.
Figures 3A, 3B, 3C, 3D and 3E show views of manifolds with different exhale valve 340 versions, and views of manifold input and overpressure gas valves, for a single patient tube connection.
Figure 4 shows a cross-sectional view of the exhale solenoid valve of Figure 3C.
Figure 5 shows measured solenoid valve force curves for a proportional solenoid valve including a ferromagnetic end stop and including a non-ferromagnetic end stop.
Figure 6 shows a manifold top view with a voice coil motor (loudspeaker) exhale valve for a single patient tube connection.
Figure 7 shows a cutaway view of the manifold in Figure 6.
Figure 8 shows a perspective exploded view of a Gas Flow Controller (GFC) module showing a portion of the calibrated volume.
Figure 9 shows a second perspective exploded view of a GFC showing the remaining portion of the calibrated volume and the restrictor.

### DETAILED DESCRIPTION

It will be readily understood that the components of the embodiments, as generally described and illustrated in the figures herein, may be arranged and designed in a wide variety of different configurations in addition to the described representative embodiments. Thus, the following more detailed description of the representative embodiments, as illustrated in the figures, is not intended to limit the scope of the embodiments, as claimed, but is merely representative of embodiments.

Phrases that mention "in one embodiment" or "in an embodiment" or the like in various places throughout this specification are not necessarily all referring to the same embodiment. A particular feature or characteristic described as "in an embodiment" may not be present in all embodiments.

Further, features, structures, or characteristics may be combined in any suitable manner in one or more embodiments. One skilled in the relevant art will see that the various embodiments described here can be operated without one or more of the specific details, or with other methods, materials, or components, or with items derived from other described embodiments, assembled to suit the required purpose.

As used herein and in the appended claims, the singular forms "a," "an", and "the" include plural references unless the context clearly dictates otherwise. Thus, for example, reference to "a database" includes a plurality of such databases and equivalents thereof known to those skilled in the art, and so forth, and reference to "the database" is a reference to one or more such databases and equivalents thereof known to those skilled in the art, and so forth. Recitation of ranges of values herein are merely intended to serve as a shorthand method of referring individually to each separate value falling within the range. Unless otherwise indicated herein, and each separate value, as well as intermediate ranges, are incorporated into the specification as if individually recited herein. All methods described herein can be performed in any suitable order unless otherwise indicated herein or otherwise clearly contraindicated by the text.

The terms "electronic circuitry", "circuitry" or "circuit," as used herein include, but are not limited to, hardware, firmware, software or combinations of each to perform a function(s) or an action(s). For example, based on a desired feature or need. a circuit may include a software-controlled microprocessor, discrete logic such as an application specific integrated circuit (ASIC), or other programmed logic device. A circuit may also be fully embodied as software. As used herein, "circuit" is considered synonymous with "logic." The term "logic", as used herein includes, but is not limited to, hardware, firmware, software or combinations of each to perform a function(s) or an action(s), or to cause a function or action from another component. For example, based on a desired application or need, logic may include a software-controlled microprocessor, discrete logic such as an application specific integrated circuit (ASIC), or other programmed logic device. Logic may also be fully embodied as software.

The term "processor," as used herein includes, but is not limited to, one or more of virtually any number of processor systems or stand-alone processors, such as microprocessors, microcontrollers, central processing units (CPUs), and digital signal processors (DSPs), in any combination. The processor may be associated with various other circuits that support operation of the processor, such as operational amplifiers, Digital to Analog Converters (DACs), Analog to Digital Converters (ADCs), Pulse Width Modulated (PWM) circuitry, wired serial communication (UART, SPI, USB) devices, radio frequency communication devices, random access memory (RAM), read-only memory (ROM), programmable read-only memory (PROM), erasable programmable read only memory (EPROM), clocks, decoders, memory controllers, or interrupt controllers, etc. These support circuits may be internal or external to the processor or its associated electronic packaging. The support circuits are in operative communication with the processor. The support circuits are not necessarily shown separate from the processor in block diagrams or other drawings.

The term "controller," as used herein includes, but is not limited to, any circuit or device that coordinates and controls the operation of one or more input and/or output devices. A controller may, for example, include a device having one or more processors, microprocessors, or central processing units capable of being programmed to perform functions. A controller may also contain analog, discrete component, and integrated circuit blocks such as amplifiers, differentiators, integrators, oscillators, control knobs, and display devices.

The term "logic," as used herein includes, but is not limited to. hardware, firmware, software or combinations thereof to perform a function(s) or an action(s), or to cause a function or action from another element or component. Based on a certain application or need, logic may, for example, include a software controlled microprocess, discrete logic such as an application specific integrated circuit (ASIC), or other programmed logic device. Logic may also be fully embodied as software. As used herein, the term "logic" is considered synonymous with the term "circuit."

The term "software," as used herein includes, but is not limited to, one or more computer readable or executable instructions that cause a computer or other electronic device to perform functions, actions, or behave in a desired manner. The instructions may be embodied in various forms such as routines, algorithms, modules or programs including separate applications or code from dynamically linked libraries. Software may also be implemented in various forms such as a stand-alone program, a function call, a servlet, an applet, instructions stored in a memory, part of an operating system or other type of executable instructions. It will be appreciated by one of ordinary skill in the art that the form of software is dependent on, for example, requirements of a desired application, the environment it runs on, or the desires of a designer/programmer or the like.

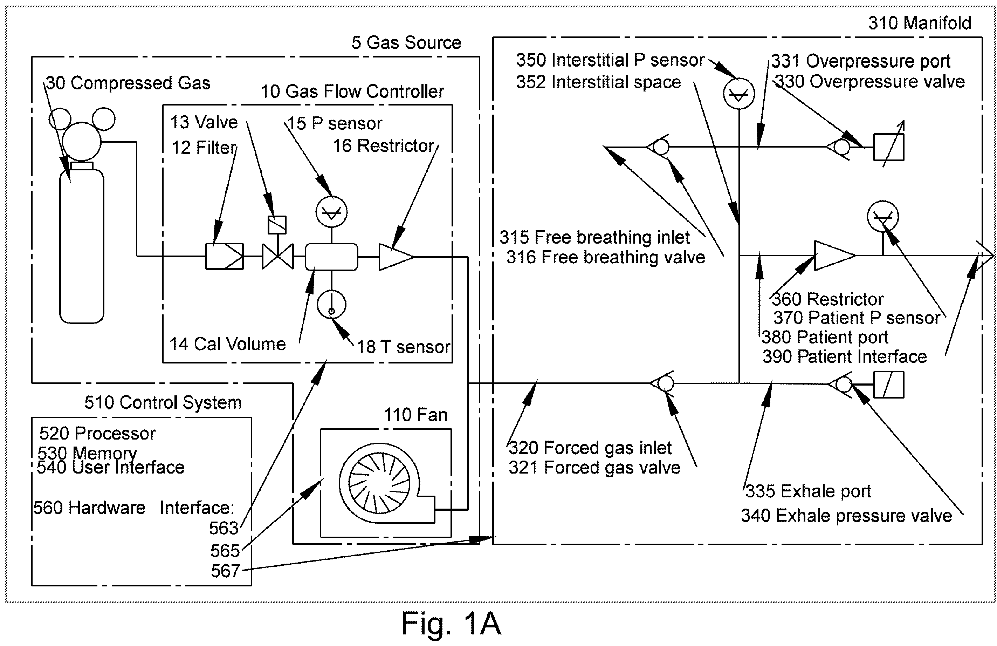

Figure 1A shows an overall schematic view of the components of an embodiment of a ventilator device or system hereof. The block 310 Manifold contains valves and ports fluidly connecting to the patient (via a patient port 380) and to a controlled source of gas via (forced gas inlet port 320). Forced gas inlet port 320 is fluidly connected to gas source 5 which contains at least one source of breathing/ventilating gas at sufficient and time-controlled flow and pressure to deliver the desired patient inhalation gas mixture. Additional ports provide control of the interstitial space pressure inside the manifold, monitored by pressure gauge 350. In Figure 1A, interstitial space 352 is represented by the vertical line descending from interstitial P sensor 350 and connecting to various valves and ports.

Free breathing inlet port 315 is normally sealed by a low-pressure one-way valve 316 and is active only when system failure and patient demand cause the valve to open and ventilate for inhalation with outside air. The optional one-way overpressure valve 330 may be attached to a further port 331 with opening pressure set higher than the normal ventilation pressure, permitting exhalation, again in case of system failure.

A number of embodiments presented herein of exhale pressure control valve 340 permit valve 340 to open upon various types of system failure, and thus may be regarded as a useful additional fail-safe mechanism, or as sufficient protection on its own to obviate the need for overpressure valve 330.

The exhale pressure control valve 340 may, for example, be operated in feedback control with interstitial pressure (P) sensor or gauge 350 to attain the desired patient inhalation and exhalation pressures. Such patient pressures may further be optionally monitored by gauge or sensor 370 through an optional restrictor 360.

The optional elements of restrictor 360 and patient pressure sensor 370 permit gas flow rate into the patient to be monitored through observation of the differential pressure drop across restrictor 360. Such flow rate measurement may, for example, be desirable as part of accurate patient monitoring. An alternative measurement of patient flow rate can be obtained through measurement of the exhaled gas from valve 340, which may similarly be accomplished using, for example, a restrictor and a differential pressure measurement across that restrictor, and subtracting that flow from the known flow rate into port 320 to yield patient flow. A further method of determining gas flow rates in any of the ports herein uses a thermal element exposed to the gas flow which exhibits increasing temperature drop or increasing thermal gradients with increasing gas flow rates. Any of such indirect differential pressure or thermal gas flow readouts can be auto-calibrated in the embodiments disclosed herein by calibrating in real time against the known inhalation flow rates provided by gas source 5, thus yielding accurate monitoring of both inhaled and exhaled gas flows.

Monitoring of patient pressure using, for example, patient pressure sensor 370 permits direct access to patient airway pressure fluctuations which can signal demand from the patient for triggering of inhalation or exhalation events. In the case of a long feed tube from patient port 380 to the patient interface 390, such fluctuations will be averaged in the volume of the feed tube resulting in attenuated patient demand signal strength. Thus while restrictor 360 may be simply the feed tube from manifold 310's patient port 380, higher sensitivity to patient demand is attained with a separate flow restriction device close to the patient interface 390, with fluidic connection of pressure gauge 370 at the patient end of restrictor 360.

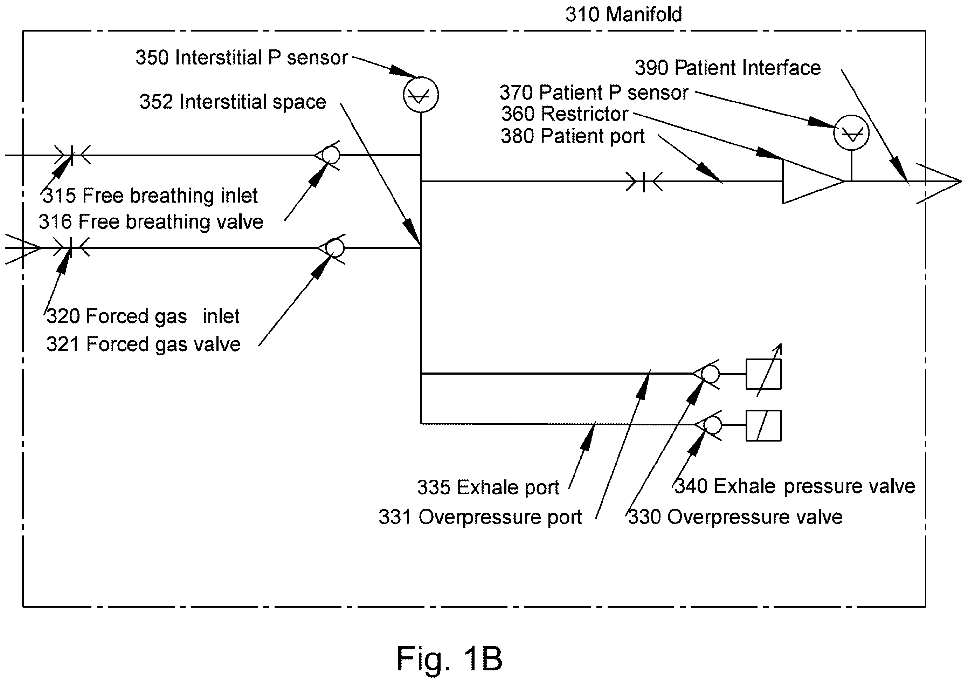

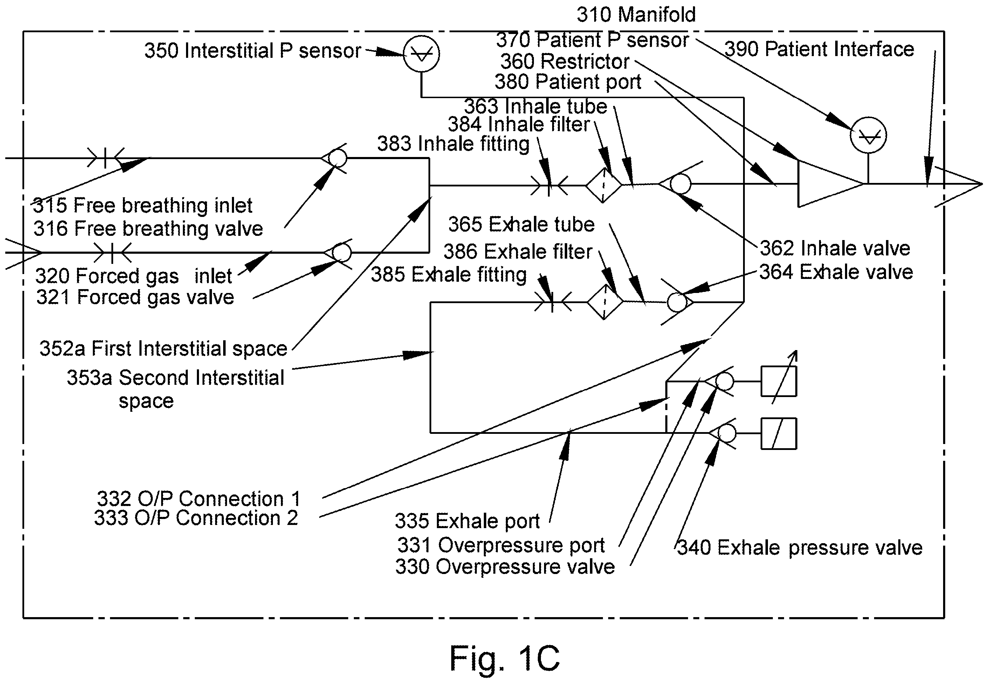

Manifold 310 may be fed at forced gas inlet port 320 by a variety of controlled gas supply sources of gas source 5. For convenience and brevity, Figure 1 shows two different types of pressurized controlled gas supply devices: a turbine or fan 110, and a Gas Flow Controller (GFC) 10. Bellows and/or pistons may also be used in generating gas flow for ventilation.

Fan 110 typically is a high-pressure blower with known pressure-flow characteristics. Such a fan would be most useful in portable applications or in resource-poor settings, where compressed gases are not easily available. Since patient gas flow rates must be monitored, and where patient flow cannot be easily measured using optional devices such as restrictor 360 and patient pressure sensor 370), patient flow can be estimated using fan characteristic curves.

Fan characteristics exhibit the highest outlet pressure P with a blocked fan outlet. As flow Q is increased by opening the blockage, outlet pressure P falls, until maximum flow is attained when pressure P, as measured relative to ambient pressure, is zero. The resultant P/Q chart thus follows a line starting at a high P axis value for Q=0, and ending at a high Q axis value for P=0. As the speed of the fan is varied by computer drive D 565 using, for example, variation of average drive voltage for brushed dc motors, or of drive frequency for 3-phase brushless dc motors, the characteristic chart line moves up at high D, and down at low D. These characteristic lines can be parameterized to yield a formula relating P, Q, drive D, - and also optionally including temperature T, which controls gas viscosity and density.

With such a formula, the known manifold interstitial pressure as measured by interstitial pressure sensor 350, and drive 565 D, yield the output flow rate. This formula-derived flow rate corresponds to patient flow if exhalation valve 340 is closed. This flow rate cannot be varied quickly, being dependent on fan rotor inertia and manifold gas volumes, so flow rate control and measurement is less responsive than a direct measure using restrictor 360 and pressure sensor 370 but is a helpful control parameter.

Alternatively, or additionally to fan 110, any number of fans, GFC 10s (Gas Flow Controllers), or other flow control systems can be employed to deliver controlled flow rates from sources of compressed gas 30 to manifold port 320.

The GFC 10 system illustrated in Figure 1A provides a simple, economical, readily sourced, and highly accurate flow control but it is evident that other types of flow controller could equally be used with appropriate calibrations to deliver flow to manifold 310.

A selection of such alternative flow controllers is described in US Patent No. 9,910,448 (C. Horwitz), and include thermal sensing 'MFC' (mass flow controller) devices which are in common use.

In addition, if dynamic control of flow rates is not required, an alternative and easily sourced restrictor approach can be used if a gas pressure regulator, or a gas pressure switch and electric valve, are available. See, for example, C M Horwitz, "Simple Calibrated Gas Feed System", Rev. Sci. Instrum. 50 (5) 652-654 (May 1979). An example of where such a simple approach is possible is in ventilation using the TCAV 'time controlled adaptive ventilation' protocol (G F Nieman et al, "Prevention and treatment of acute lung injury with time-controlled adaptive ventilation: ...", Ann Intensive Care (2020) 10:3 DOI.org/10.1185/s13613-019-0619-3). In TCAV, a constant and limited flow of fresh gas may be applied to port 320, with pressure dynamically controlled by valve 340, and with occasional and brief openings of valve 340 to exhaust gases, including waste gases, to the ambient. As will be shown below however, the benefit of this pre-calibrated gas flow method, which is subject to fouling and regulator unreliability and is not able to be modulated in the case of a static gas pressure regulator being used upstream of the restrictor, may not be sufficient to favor this over the GFC method.

While a plurality of possible gas flow control devices from source 30 to port 320 is thus available, below the detailed operation of a representative embodiment of GFC module 10 is given. This embodiment of GFC module 10, which is, for example, illustrated in Figures 1A, 8 and 9, offers precise auto-calibrated and adjustable gas flow with a minimum of components. Gas at a higher pressure than is to be required by calibration volume 14 and restrictor 16 for the desired gas flow into port 320 enters through optional filter 12, and flows into volume 14 when valve 13 is opened to a predetermined state, which may be the fully open state, or may be some intermediate state if a proportional valve is employed.

Volume 14 will to some extent average the resultant pulsations of gas flow from valve 13 and provide a sufficiently constant flow of gas through restrictor 16 to port 320. Such gas flow pulsations may yield pressure variations in volume 14 larger than 60% of its average pressure, or smaller than 0.1% of its average. The resultant flow variations are further averaged by manifold volume in its interstitial space as measured by Pressure sensor 350, and by patient tubing lines from port 380 to patient interface 390. Thus, even large pressure pulsations in volume 14 may not be apparent to the patient or clinically significant.

However large or small these pressure pulsations are, GFC system 10 may employ rate of change of pressure or low and high point pressures to accurately determine flow rate, using the ideal gas law PV = nkT, where P is the pressure in a volume V, at temperature T, which encloses the number of molecules n, and where k is the Boltzmann constant as further described in U.S Patent No. 9,910,448. For example, the rate of change of pressure dP/dt (where t is time) directly yields dn/dt, the molecular rate of gas flow, when valve 13 is closed. Temperature sensor 18 permits correction for absolute temperature T in the above formula, yielding precise flow rate measurement independent of ambient temperature variation.

An example of a temperature sensor suitable for use herein is a simple two-terminal silicon diode with a small dc bias current bolted onto the GFC metalwork, whose forward voltage varies with temperature and is easily monitored. Many other types of temperature sensors may be employed such as thermistors, temperature-dependent resistors, and integrated circuits.

In the present application of a GFC flow control to medical ventilators, control of instantaneous gas flow rate is not as critical as in some semiconductor process applications, where often control and instantaneous stability within 0.1% is considered a desirable characteristic. Here, large pressure pulsations in volume 14 may be averaged in patient tubing and lungs to yield a substantially constant patient pressure and flow. As an extreme example, the pressure in chamber 14 may be permitted to pulse to a high pressure Ppk, then fall to ambient pressure on each cycle of valve 13, if volume 14 and desired flow rates are sufficiently small. This would be described as 100% pressure cycling of the peak pressure in volume 14. This large pressure variation, with possible flow cessation between gas pulses, still permits accurate average flow rate measurement using pressure differences at the beginning and end of a pulse, rather than through monitoring dP/dt, the instantaneous rate of pressure fall during a pulse.

For any level of pressure cycling, with gas pulses from volume 14 cycling in pressure from Ppk to Pmin, the average molecular flow rate Q is simply calculated from volume 14 [V], its absolute pressure difference during a pulse [Ppk - Pmin] and the pulse frequency f, as Q = f * n = f * [ Ppk - Pmin ] * V / [ kT ] . Here n is the number of molecules transferred from volume 14 on each pulse. Hence accurate gas flow rate control can be attained without needing to monitor rates of pressure fall in chamber 14. For example, a simple oscillator with known frequency f combined with pressure difference monitoring is sufficient. As an example, one test setup employed valve 13 cycling frequency f = 5Hz in a GFC with inlet air pressure and Ppk of 100psi, volume 14 pressure modulation of approx. 30%, and a volume 14 of 60cc feeding a flow-averaging patient line of length 1.2m and inner diameter 20mm, connected to a 500cc test lung volume. The resultant patient pressure waveforms had less than 10% gas flow pulsation ripple.

While many existing ventilator designs aim to attain smooth flow waveforms, the slight pulsatile waveform inherent in the above example and disclosures herein may be either enhanced by the use of a small volume 14 relative to the patient tubulation and lung volume, or suppressed if a relatively large volume 14 is employed. An alternative to alterations of volume 14 size would be to modulate valve 13 conductance, as described below, to yield the desired pulsatile flow waveform through control of Ppk. Patients with ARDS and lung congestion due to viscous fluid buildup as described by Nieman (op cit) have been shown to benefit from a gentle oscillatory pressure waveform. One mechanism that can explain some of the benefit of oscillatory patient flow in the case of fluid-congested lungs is "fluid thread breakup" wherein slight vibrations induce disturbances in fluids which can enhance the rate of thread breakage. See, for example, Javadi et al, "Delayed Capillary Breakup of Falling Viscous Jets, Phys. Rev. Lett. 110 (14), 144501, DOI: 10.1103/PhysRevLett.110.144501 (2013). Hence the devices, systems and methods hereof afford clinicians an additional tool with which to address difficult patient requirements and high morbidity rates.

In any application of the GFC concept, the instantaneous flow rate through restrictor 16 is related to the absolute pressures at its inlet P1 and outlet P2. For example, in the case of a tubular restrictor operating in laminar flow, flow rate Q is proportional to a constant dependent on restrictor geometry multiplied by [ P1 - P2 ] * [ P1 + P2 ] = [ P1 * P1 ] - [ P2 * P2 ]. Other flow conditions (for example, supersonic flow through an aperture, or high-rate turbulent flow in a tube) yield other pressure-flow relationships. With such knowledge of restrictor conductance properties, a fast flow response can be achieved before waiting for the first pressure cycle to be completed. The average pressure P1 in volume 14 required to deliver the desired flow will be known in advance. Valve 13 can then be controlled to rapidly attain at least that pressure, yielding approximately the desired flow on the first pressure pulse.

Subsequently, rates of pressure fall or pressure difference measurements in chamber 14 yield updated calibrations of restrictor 16 properties which can then inform subsequent pressure cycles of what pressure ranges to aim for. In addition, a historical record of restrictor 16 properties, as would be retained in a computer monitoring and control system or manually in the case of manual control, yield helpful information on the condition of the restrictor, permitting advance warning of the need for cleaning or replacement in the case of partial fouling. Such continuously monitored restrictor conductance thus not only yields rapid attainment of desired flow rates, but also preventive maintenance information, while retaining the inherent self-calibration and high accuracy of the GFC method even while restrictor conductance properties may be changing.

Since pressure pulsations in volume 14 occur throughout the gas flow time, they provide a direct and real-time calibration of flow rate based upon fundamental principles, as long as calibration volume 14 is known. This removes the need for specially calibrated adjustable valves, pressure regulators, known restrictor elements, calibrated flow sensors, or other indirect flow control devices as are common in the art.

A small volume 14 with its consequent short response time yields benefits, since shaped flow waveforms are sometimes required. Typical response times in ventilators which require shaped flow waveforms are of the order of 0.3 seconds, with inhalation times ranging from 0.8 seconds to 7 seconds. Flow rates during inhalation range from 5 to 90 liters per minute, all depending on the patient size and the protocol in use. To meet this wide range of operating conditions and short response times, attention to the detailed design of valve 13 is required.

For any given set of conditions including a given valve 13, delivery pressurized source/tank 30, and desired flow rate into port 320, a small volume 14 will yield correspondingly large pressure fluctuations or require faster cycling of valve 13. If valve 13 is able to be cycled quickly, it is able to provide rapid modulation of the pressure waveform and render the average pressure, hence flow, more constant, yet more rapidly adjustable, for a given size of calibration volume 14.

One alternative to fast operation of valve 13 is a proportional valve 13, capable of being opened to a varying degree. From the fully off position it may be cracked open sufficiently to let a small amount of gas into volume 14, then closed again. Clearly if the inlet pressure from source 30 is far higher than that in volume 14, a smaller fractional opening yields correspondingly smaller pressure peaks for a given valve opening time.

A further alternative would be paralleling of valves (or providing a plurality of valves connected in parallel) to form a composite valve 13, with a small fast-acting valve capable of perhaps 3ms actuation time supplying the lowest flow requirements, placed in parallel with one or more larger valves, each with slower actuation times but with larger flow capability. The actions of such on/off types of non-proportioning valves 13 can be understood more fully by considering the action of valve 13 in various flow regimes, given a relatively high and fixed source gas pressure 30.

At the highest flow rates required, valve 13 may be fully open for a large fraction of time, closing for brief periods to both regulate to the desired pressure in volume 14, and to monitor the absolute flow rate. Thus, there would be relatively small fluctuations in volume 14 pressure, and thus small output flow fluctuations. In this case, the series conductances of valve 13 and its associated piping, combined with the known incoming as 30 pressure, yield a large "on/off" time ratio.

In contrast, at the lowest desired flow rates which would be appropriate for neonatal pediatric ventilation, a relatively low pressure in volume 14 is required to deliver low flows through restrictor 16. Thus, if valve 13 were to open fully, the high surge flow from source 30 and resulting steep rate of pressure rise in volume 14 requires a very short valve 13 opening ("on") time, and a relatively long closed ("off") time. Valves with large orifices and conductances are required to deliver the highest adult-level flow requirements, but their correspondingly large armature inertia yields a lower response speed in comparison with small valves, hence in a greater difficulty in delivering at the lowest flow levels if the small on/off time ratio results in unacceptable low-flow pulsations at patient interface 390.

Hence a non-proportioning valve 13 may comprise a plurality of paralleled valves with their corresponding conductances ranging from low to high, and corresponding operable speeds ranging respectively from high to low, yielding a wide range of low-pulsation patient flows, and so simulating proportioning valve action in a stepwise manner by changing between valve orifice sizes instead of between proportional valve states.

Fail-safe operation of medical devices is a desirable characteristic and the above plurality of valves yields a further degree of such protection. While a "stuck-open" valve failure is protected against by overpressure valve 330, parallel valving permits continued operation in the event of a valve failing to open, due possibly to fouling, a burnt-out coil, a failed drive transistor, or a broken wire. In such events, less optimal performance may be maintained while an alarm may be sounded.

### SYSTEM MONITORING and CONTROL

Computer control 510, incorporating standard analog interface blocks such as amplifiers, digital to analog and analog to digital converters, pulse width modulated outputs and power supplies, is an integral part of a number of representative embodiments hereof.

Fan control 565, as described in the details to Figure 1A, may, for example, require use of a formula calculation to obtain the correct fan drive level for a given ventilator status. In combination with setting the desired interstitial pressure 350 by feedback control and cycling those controls while monitoring gas flows to the patient, ventilation according to various protocols can be achieved. Additionally, if the GFC gas supply system is chosen, the rate of change of pressure in volume 14 yields a direct measure of the gas flow which can be readily modulated or terminated using valve 13. For example, if constant volume for every inhaled breath is desired, the integrated flow over inhaled time yields volume, and while holding the inhaled pressure waveform to the desired shape, when the desired volume trigger point is reached inhalation can be terminated by shutting valve 13. If a fan is used, one would tum off fan 110, with backflow prevented by valve 321. During the exhalation phase, valve 340 is controlled to maintain the desired PEEP pressure level. If constant pressure ventilation is needed, a simple time - pressure waveform is applied, but without triggering cycle cessation on the accumulated volume in each breath.

Modes of operation used commonly in the ventilator community (which go by various acronyms) can all be supported by the devices, systems and methods hereof; a selection of which can be found in Neiman et al (ibid).

In addition, there are many system parameters, and derived parameters, that are clinically useful, all of which can be monitored and derived using the devices, systems and methods disclosed herein.

A typical ventilator control suite permits clinicians to choose between patient-triggered breath provision for the conscious patient, or mechanically triggered breathing for the paralytic patient, or a combination thereof; and inside those broad categories they can choose pressure, volume, tidal volume, inhalation time, exhalation time, volume vs. time waveform shape, breathing rate, maximum flow, inhalation pressure, PEEP level, breathing assistance trigger sensitivity, pressure support level, and other parameters for more modem ventilator protocols (Nieman et al, ibid). Alarms, controls, and displays also include pressure support time limit, low peak pressure, high pressure, high breath rate, high tidal volume, low minimum volume, manual breath, inhalation time, inhalation/exhalation hold, and current flow rate and pressure. Controls are also present for oxygen and other gas mixing ratios, and there can be a display for the measured oxygen mix fraction. All of these controls and displays can be provided by the devices, systems and methods hereof via monitoring, and control of, pressures, flows, and integrated flow volumes.

If monitoring of lung elasticity is required in the constant volume mode, an inhaled breath cycle can be held such that flow is held at a low level, to observe how the pressure changes with time; or one can observe transient pressure changes as the lung is filled to get another measure. Alternatively, after dumping gases briefly in the TCAV method one can observe the slope of gas flow and the pressure curve shape when restoring steady inhalation pressure. There are many other parameters that can be measured and logged, corresponding to the chosen ventilation protocol and lung condition.

A typical range of internal data update periods in hardware or communication interface 560 is between 3ms and 100ms depending on the update period required by the parameter in question, with critical analog signals being able to be measured at update periods as short as 1 microsecond. Since such rates of measurement and control of flows and pressures are at high speed in relation to the timings of ventilation cycles, all associated relevant parameters from system measurements and calculated derived parameters can be logged.

In addition, computer control 510's user interface 540 (which may convey information via audio, visual and/or tactile means) permits clinicians to see trends and respond to them in real time, whether they develop over a few minutes or over a few weeks. Thus, the logged output is available in graphical form with adjustable plotted parameters, and an adjustable time axis. Such logged data can also be downloaded over a network or into a memory device to enable storage of patient records elsewhere.

Alarm settings in computer control 510's user interface 560 enable clinicians to be warned of faults, incipient maintenance needs, and of patient parameters going out of set limits. In particular, preventive maintenance needs for the GFC system can be alerted far in advance of needing maintenance attendance, permitting action to be taken before harm to patients occurs.

Figure 1B shows a detailed view of manifold 310 in the case of a single patient breathing tube connection. The components shown in Figure 1B are the same as those shown in Figure 1A, but a number of components are moved to group the inhale and exhale valve functions and flows. In cases where patient lung volume permits rapid exhaustion of gases stored in the patient breathing tube, one line is sufficient and simplifies both manifold and patient plumbing. There may be a bottle installed in the patient breathing tube to collect liquid ejecta from the patient.

Figure 1C shows a detailed view of manifold 310 in the case of a dual-tube patient breathing connection, which is commonly employed by 'patient circuit kits' used for ventilation or anesthesia. Such kits may include one-way valves 362 and 364 at the patient intubation connection (patient interface 390) and may include provision for bottle collection of liquid ejecta along the exhalation tube or line. The manifold components shown are generally the same as in Figure 1A and Figure 1B, but with the splitting of interstitial space/section 352 into an first or inhalation space 352a and a second exhalation space/section 353a. Overpressure valve 330 is configured to vent excessive patient pressures and could be placed in parallel with exhale pressure valve 340 for simplicity of plumbing in cases where valve 364 is in the vicinity of the patient; or between valve 364 and the patient if valve 364 is incorporated in the manifold. Such possible connections are shown as overpressure (O/P) connections 333 and 332 in Figure 1C, respectively. In between these two connection points is shown the one-way exhale valve 364.

Typically, disposable filter 384 for inhale tube/line particles and disposable filter 386 for exhaled microorganisms are placed at the ventilator to protect the patient and the ventilator, respectively, against cross-contamination. These filters may be attached respectively to fittings 383 and 385.

Monitoring of interstitial pressure with sensor 350 may be achieved most accurately, and with the economy of using only one pressure sensor, by connecting to patient port 380 as shown, but if valves 364 and 362 are at the patient intubation connection, this may require a separate pressure monitoring tube, as would the patient P sensor 370. Thus two breathing tubes (exhale and inhale), plus two smaller-diameter tubes for pressure monitoring would need to be connected at patient interface 390.

An alternative to running all four tubes to patient interface 390 would be to place the connection for sensor 350 in second (exhalation) interstitial space 353a, whereby it would monitor the required pressure waveforms during rising patient pressures during inhalation through the action of one-way exhale valve 364, as well as exhalation pressures. It would however not monitor inhalation pressure cycles on their downward or steady cycle portions. If such monitoring is required, a further pressure sensor should be mounted such that one sensor is attached to first (inhalation) interstitial space 352a and a second sensor to second (exhalation) interstitial space 353a, requiring a total of three pressure sensors, including optional sensor 370.

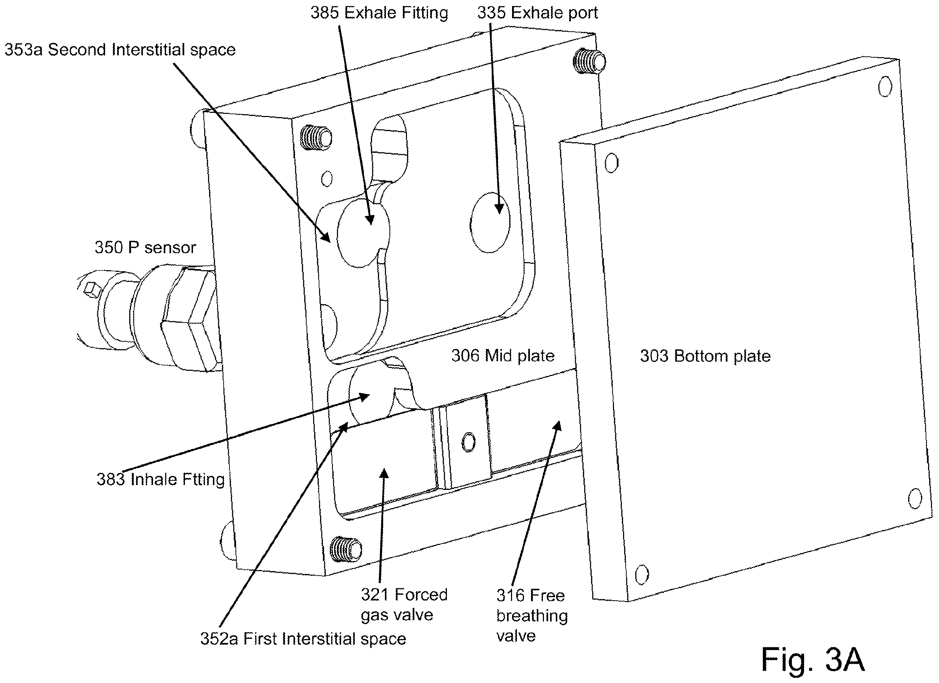

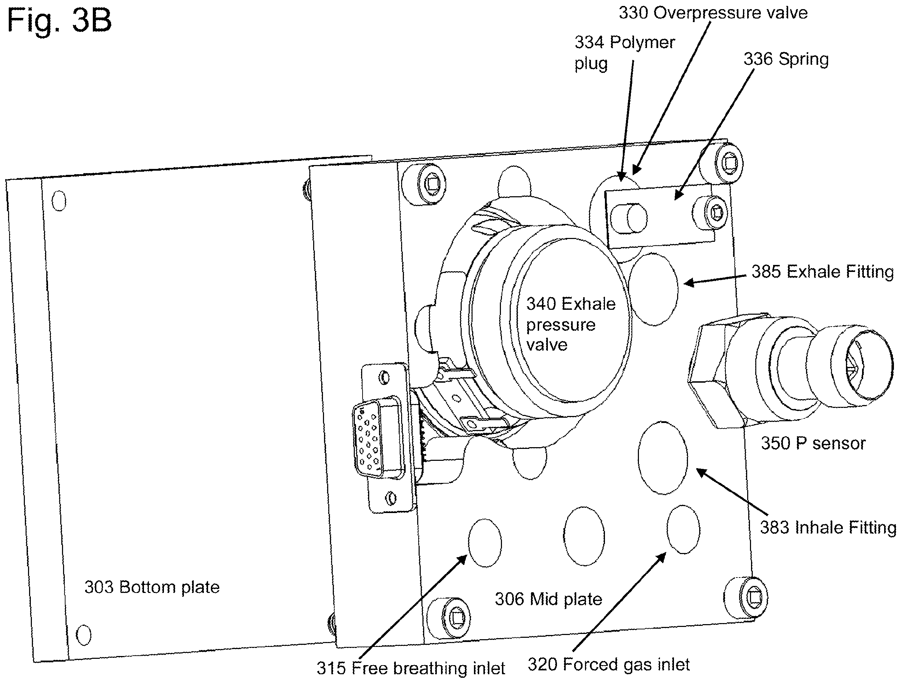

Some of the later figures in this disclosure for simplicity only illustrate the simpler case diagrammed in Figures 1A and 1B. Those skilled in the art would be able to split the illustrated single patient port 380 into two separate ports, as is shown in Figure 3A and 3B illustrating that more complex case diagrammed in Figure 1C, connecting to two separate interstitial spaces, and with two separate pressure monitoring ports instead of one.

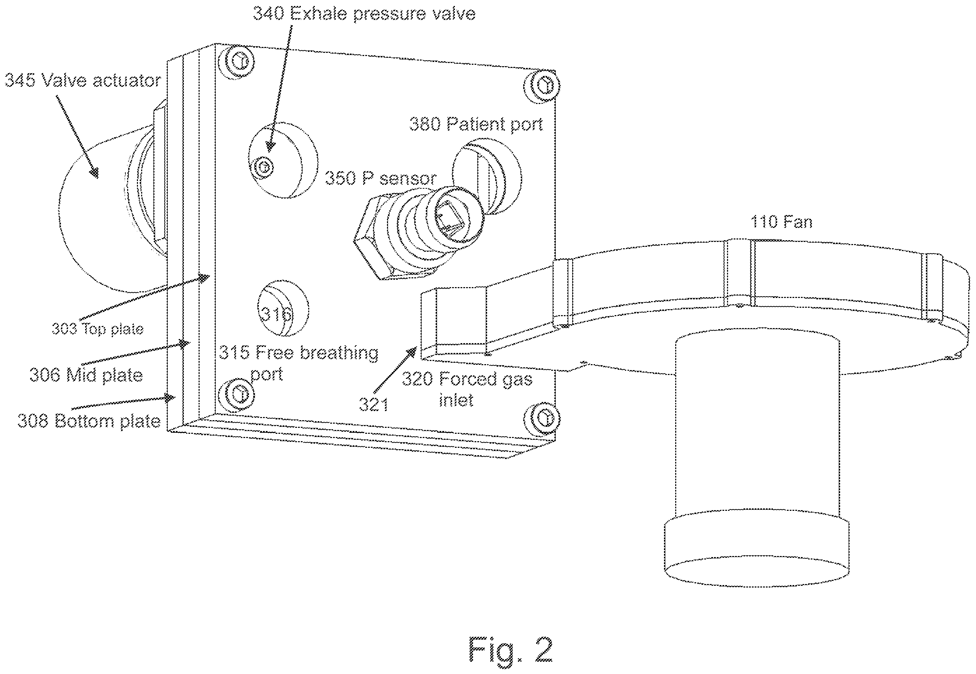

Figure 2 shows how a single air source using fan 110 for feed to port 320 may be installed on a manifold using a solenoid coil driver 345 for exhale pressure valve 340. This manifold may be constructed from top plate 303, mid plate 306, and bottom plate 308 composed of generally flat plates, modules or blocks of 100mm x 100mm pieces of PVC polymer bolted together at their corners in a number of representative embodiments. Such plates may, for example, be sufficiently flat to form a seal when connected directly or via an intervening sealing member (for example, an elastomeric or other sealing member). Any other material capable of sealing against gas leakage at the relatively low pressures present here, between the bolted surfaces to the extent required in this application, such as poly methyl methacrylate, polypropylene, aluminum, steel, etc. would be suitable for manifold construction. The chosen material should have its thickness selected to avoid bending-induced leakage at pressures of approx. 10,000 Pa of more than 1 standard liter per minute (slpm) flow rate. Four bolts are shown holding all three plates together. If using thin materials, additional bolts may be required to attain this leakage level. Optionally, an elastomeric or other seal between the plates could be used to maintain a low leak rate. Pressure sensor 350 communicates fluidly with the interior passages in interstitial space 352, with patient port 380, exhale valve port 335 to exhale pressure valve 340, forced gas inlet port 320 via forced gas valve 321 and free breathing port 315 via free breathing valve 316.

Figures 3A and 3B show a similar manifold with a voice coil actuator exhale pressure valve 340. A voice coil actuator, which is also known as a non-commutated DC linear actuator, is a type of direct drive linear motor. The term "voice coil" is derived from one of the first applications of such actuators in vibrating the paper cone of a loudspeaker. Voice coil actuators may be used for a wide range of applications, including moving larger masses. Voice coil actuators include a permanent magnetic field assembly (including permanent magnets and ferrous steel) as well as a coil assembly. When current is caused to flow through the coil assembly, it interacts with the permanent magnetic field and generates a force vector perpendicular to the direction of the current. That force vector can be reversed in direction by changing the polarity of current flow through the coil.

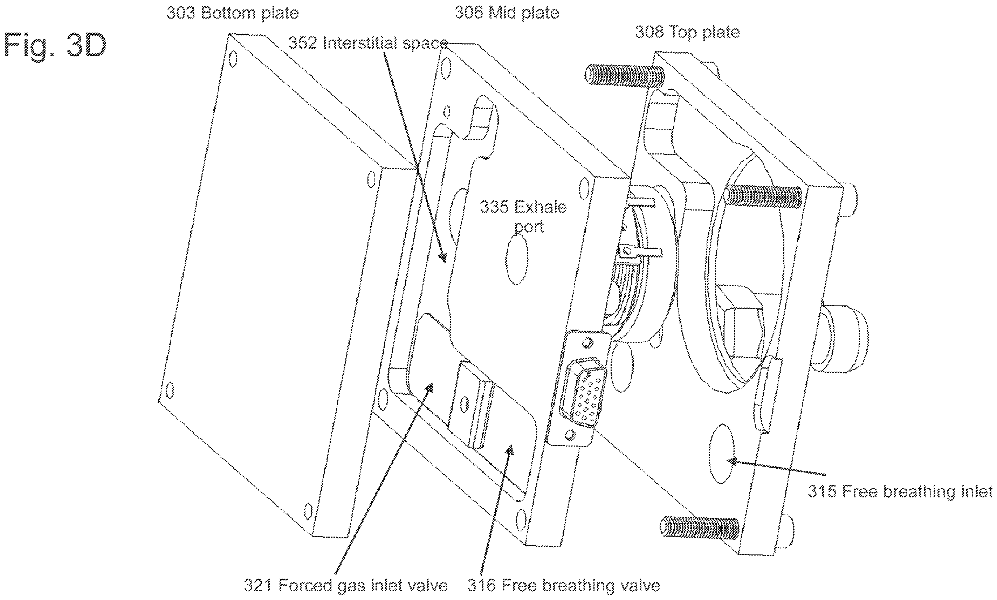

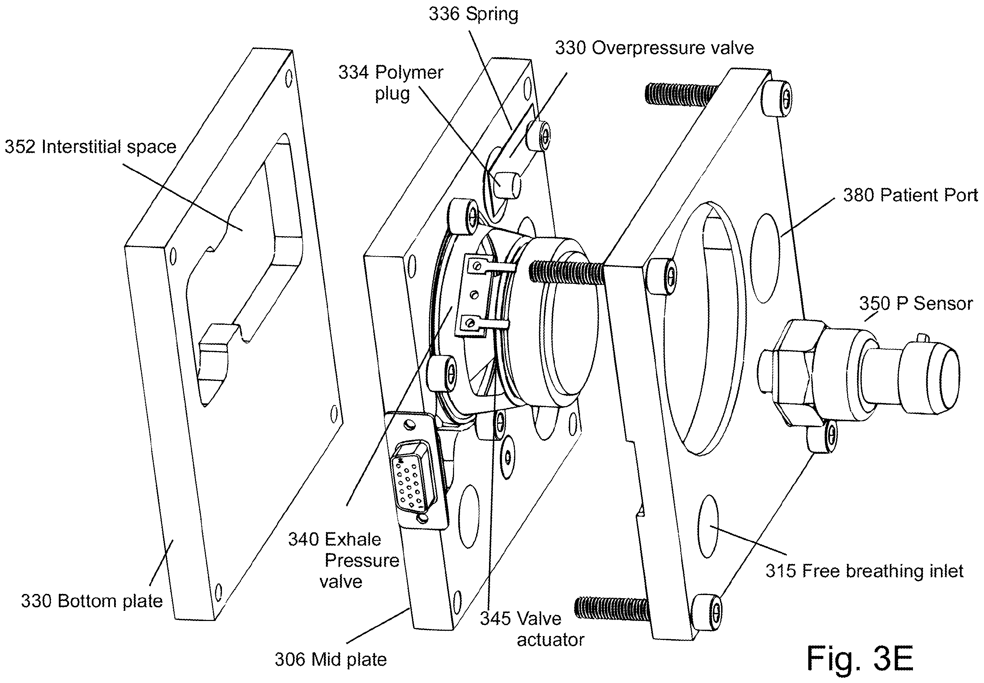

Illustrated in Figures 3A and 3B is first (inhalation) interstitial space or section 352a communicating or in fluid connection with valves 321 and 316, which may, for example, be constructed of simple flaps that close with first (inhalation) interstitial space 352a pressure higher than at their respective ports, which would occur during exhale cycles, and open when gases are to be admitted into first interstitial space 352a. The patient receives inhalation gas flow from inhalation port or fitting 383 as in illustrated in Figure 1C. In the case of a single breathing tube, the patient receives inhalation gas and expels exhalation gas via a single patient breathing tube connected to a single patient port 380 as illustrated in Figures 1A, 1B, 3D and 3E.

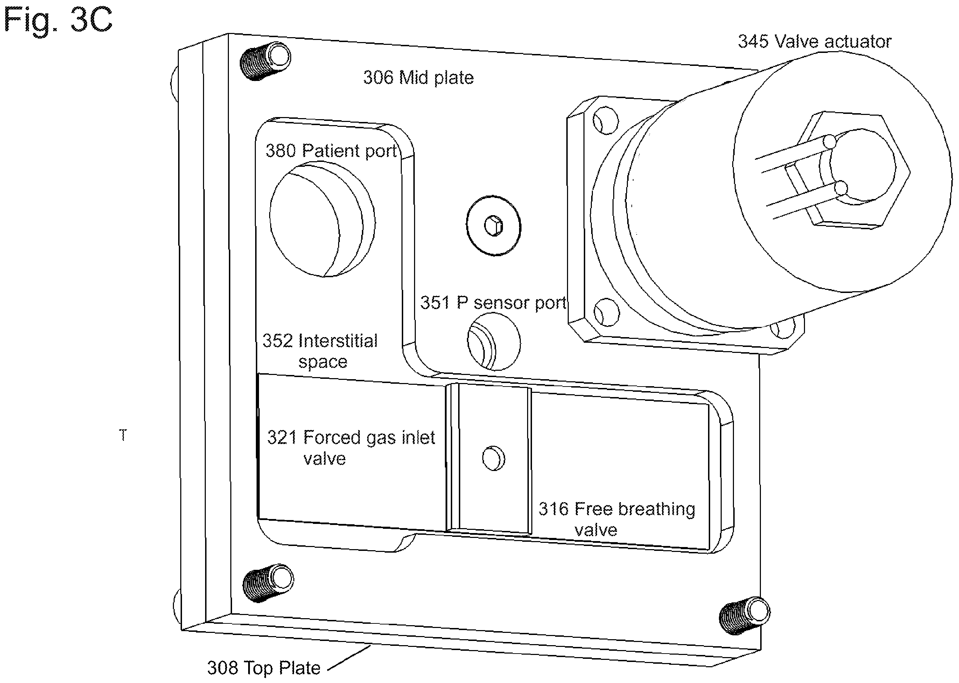

The exhale cycle through exhale valve port 335 includes gases from patient port 380 and interstitial space 352 in the case of a single patient breathing tube. In the case of a dual breathing tube, the exhale cycle through exhale valve port 335 includes gases from patient exhale port or fitting 385 which feeds into second interstitial space 353a. [Exhale valve port 335 communicates fluidly with interstitial space 352 (or second interstitial space 353a), which may, for example, be channeled in bottom plate 303 to exhale valve port 335 (as required with the thin plates shown in Figures 2 and 3C), or channeled in mid plate 306 in the case shown in Figures 3A and 3B, which illustrate a sufficiently thick mid plate 306 to not require a top plate 303, or bottom plate 303 channeling.

Overpressure valve 330 also communicates fluidly with (overpressure valve) port 331 and interstitial space 352 in the case of a single breathing tube, or as described in Figure 1C, to one of the two possible attachment points in the case of dual patient breathing tubes, permitting exhalation in case of failure of system controls or seizure of exhale pressure valve 340. Overpressure valve 330 is shown in Figure 3B as polymer plug 334 sealing against an orifice and held in place by spring steel 336. For the geometry shown, spring steel 336 is approx. 10mm wide, 25mm long, 0.25mm thick and yields to overpressures of approx. 10,000 Pa.

The purpose of top plate 308 shown in Figures 2, 3C, 3D and 3E is to enable port fittings to be attached without complicating assembly of mid plate 306, to protect overpressure valve 330, and to port all exhaled gases into a single orifice for venting appropriately. However, for further simplification, pressure sensor 350 could be mounted onto mid plate 306 or onto bottom plate 303, with port fittings connected to mid plate 306, as illustrated in Figure 3A and 3B. The resultant structure would then be comprised of two easily machined plates and a minimum of additional components.

Figure 3C shows a view of a manifold similar to that shown in Figure 2 with a removed bottom plate 303. The one-way valves 316 and 321 are shown covering ports 315 and 320, respectively and include, in the illustrated embodiment, flaps of any suitable material such as steel foil, PET polymer, CTFE polymer, laminated card stock, or rubber. With minimal pressure on their respective ports, they open to permit inward flow of gas. Pressure sensor port 351 communicates fluidly with the interstitial space 352 via a hollowed-out region in bottom plate 303 (not shown).

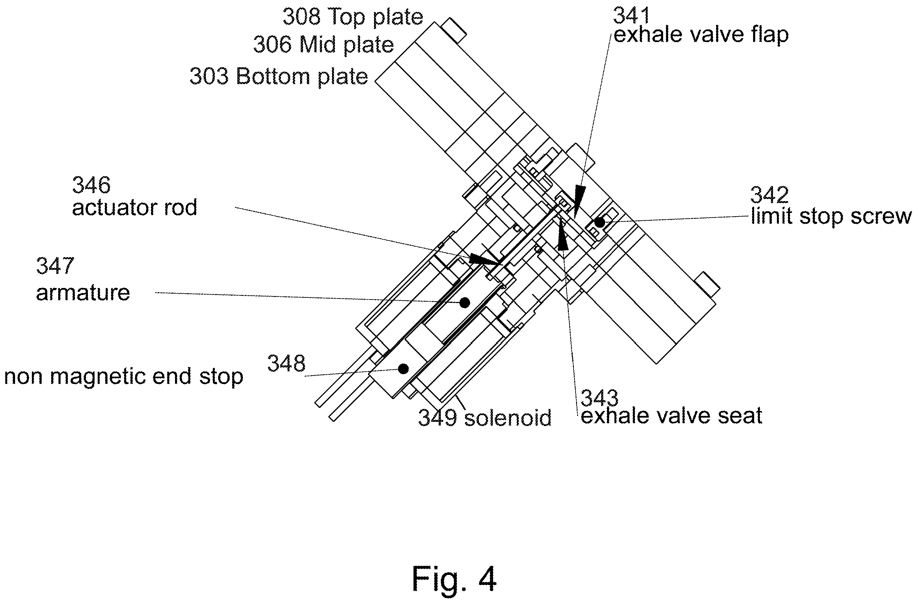

Figure 4 shows a sectional view of the solenoid drive mechanism from Figures 2 and 3C for exhalation valve 340. This valve, in either of the configurations of Figures 3A and 3B, or Figures 2, 3C, and 4, provides important functions since it not only controls exhalation pressure, but also must hold against, and perhaps adjust in real time faster than the GFC or fan gas supplies can do, the inspiratory pressure applied to the patient line. Thus, this valve is in constant use, and is controlled electronically with feedback. Such feedback is mainly derived from interstitial pressure sensor 350, with additional feedback as needed from optional patient pressure sensor 370 to maintain the desired patient pressures at all times.

Exhale valve flap 341 is prevented from over-excursion by limit stop screws 342 mounted in top plate 308. In the other direction, flap 341 seals against the surface machined into 306 mid plate, in its exhale valve seat 343 region. Actuator rod 346 is mechanically attached to exhale valve flap 341, and also to armature 347 which is constructed of a ferromagnetic material. In a number of embodiments, when manifold 310 is mounted with bottom plate 303 at the bottom, gravity ensures that armature 347 and flap 343 bear downwards pressure on exhale valve seat 343, providing a small minimum exhale valve static pressure before the valve allows interstitial space 352 (or second (exhalation) interstitial space or section 353a) to be vented through this exhalation valve 340. In such an embodiment, the orientation of actuator valve 345 with respect to the field of gravity is important. In case of power failure this provides a fail-safe, mechanism permitting free breathing, which may be supplemented by overpressure valve 330 (an option shown only in Figure 1A). The material of 341 exhale valve flap may be any suitable material, including those mentioned for the one-way valves 316 and 321 in Figure 3A with the likely exception of materials such as laminated card stock which may take permanent bends and fail to re-seal.

Exhale valve seat 343 is shown in Figure 4 to be counterbored in the surface middle plate 306 facing bottom plate 303, to ease gas flow through this exhale valve 340. The valve port diameter is preferably small to enable easy actuation of valve 340 with minimal electric current and solenoid 349 heating, thus with increased system reliability. However small port diameters, with their high flow resistance, yield high exhale backpressures. While ventilator protocols such as PEEP with high exhale backpressures are common, to accommodate all possible protocols the minimum exhale backpressure should be kept low. In a representative embodiment, we have found that the illustrated approx. 8mm diameter can yield acceptably low exhale backpressure under the following conditions. Further, in that representative embodiment, we counterbore to yield a short airflow distance at the seat diameter and assure 3mm or more of clearance between exhale valve seat 343 and exhale valve flap 341 when flap 431 is in its fully open position. Other combinations of diameters, counterbores, and open flap distances can be arranged by those skilled in the art to achieve similar results.

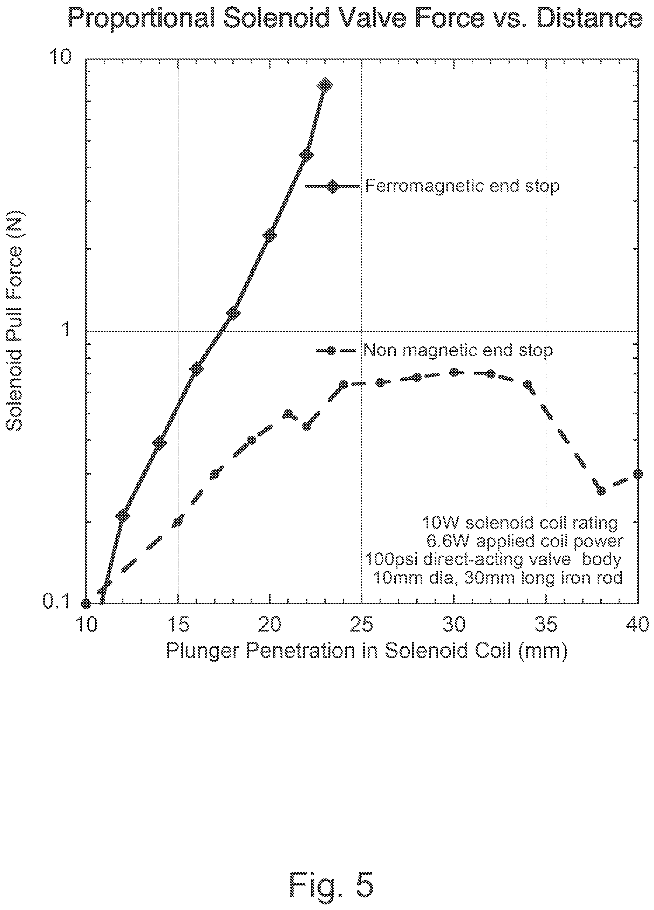

Of note is that the solenoid valve actuator, with magnetic field provided by solenoid 349 on armature 347, is in this implementation a standard direct-acting pneumatic valve with a 10W coil and nominal 100psi maximum operating pressure, but with modifications. One change is to the valve seat, which is removed to permit armature 347 to move a greater distance away from solenoid 349 than is normal. Another change is the attachment of actuator rod 346 to armature 347, preferably in a manner that permits easy removal for adjustment and maintenance such as a threaded and screwed connection, but also in a manner that prevents rotation and winding-out during operation, such as with a backing nut, thread locking compound, or glue. A further change is in the end of the stainless-steel housing tube in between solenoid 349 and armature 347. In this case the standard housing was terminated in a ferromagnetic end stop, which may be replaced with a nonmagnetic end stop 348. The effects of making this optional change are shown in Figure 5.

Figure 5 shows force vs. distance curves for armature 347 immersed to different depths inside solenoid 349. The ferromagnetic end stop curve shows higher forces at nearly all positions than for non-ferromagnetic end stop 348, with sharper divergence as armature 347 approaches the end stop at approx. 24mm. While higher forces may be helpful, in the current case we desire easy electronic feedback control of valve position to enable pressures in interstitial space 352 to be modulated with good transient response. In such a case, electronic feedback loop stability is easier to adjust where elements in the feedback loop exhibit approximately linear responses. Here, forces with a non-ferromagnetic end stop, though lower, remain roughly constant over a 10mm range, making proportional control and mechanical adjustments simpler.

However in cases where a higher force is desirable, such as where very high inhalation pressures are deemed necessary or where a lower exhalation valve backpressure when open is needed implying a greater than normal travel distance, the ferromagnetic end stop yields forces ranging over a factor of approximately 2, and overall forces higher than in the non-ferromagnetic case by a factor of between 2 and 4, with armature 347 travel constrained to approximately 3mm. This clearly requires careful adjustment of actuator rod 346 length and a feedback circuit design tolerant of loop gain variation, generally implying a more limited high-frequency response.

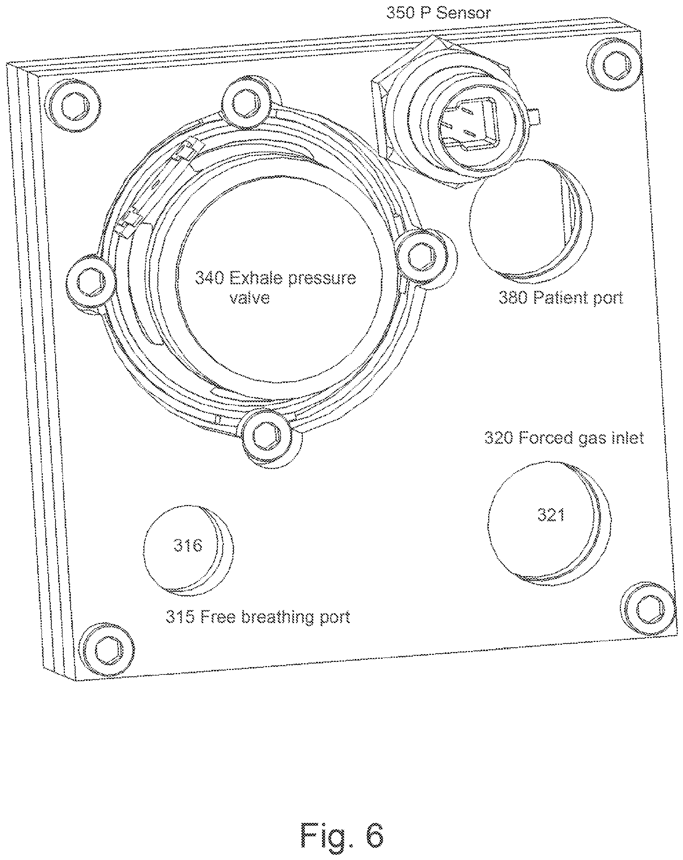

Figure 6 again shows the simple voice coil actuator form of exhalation valve 340. Instead of using a solenoid valve intended for high-pressure gas control, many other forms of valve actuation can be envisaged for this ventilator application working with a differential pressure of at most 1/10 of atmospheric pressure.

Another representative example of a valve actuator suitable for use herein is an arcuate-movement motor used to drive read/write heads across magnetic hard disk drive surfaces. Such devices use a "voice coil" pivoted between rare earth magnet slabs. These can provide 5N force over a wide actuation distance with only 500mA of voice coil current. This is far more force than is needed, or is available, from the solenoid in Figures 2 through 5, but with a more difficult mechanical implementation.

Alternatively, a standard linear voice coil motor may be applied. Such devices are readily available (see, for example, http://moticont.com/pdf/lvcm-all.pdf). One such actuator that is readily available in both in the developed and less-developed parts of the world is audio loudspeakers. Some such actuators may have more than 3mm travel and a sufficiently hard central dome to form a reliable form of exhale valve 'flap' 341. With forces more than sufficient for this application, such loudspeakers or their equivalent linear voice coil motors can be easily integrated into this manifold design. With the 52mm diameter speaker shown in Figures 5 and 6, rated at 5W peak, drive at 4W yielded 1.5N force, double what is needed in the design illustrated here to control at the highest anticipated ventilation pressures.

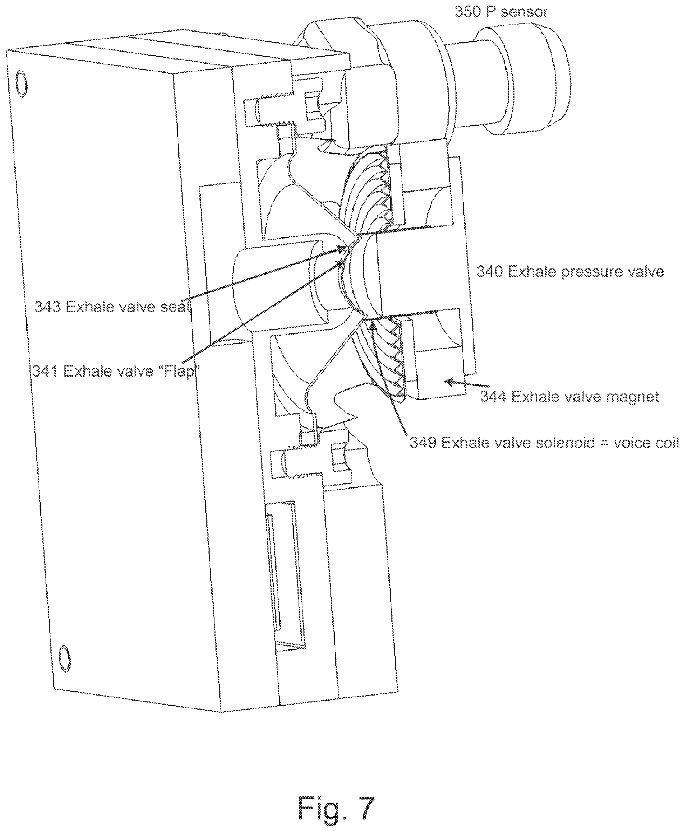

Figure 7 shows a cross-sectional view of the seal area for the above-described voice coil or audio loudspeaker implementation. Visible are valve seat 343 brought up to the height of exhale valve 'flap' 341 attached to solenoidal voice coil 349, which rides in and out of the exhale valve magnet 344's pole pieces, and is supported by a sprung corrugated member attached to the outer housing as well as by the conical surface at the loudspeaker front.

Referring to the plan view showing this loudspeaker mounted on bosses with four screws in Figure 6, around those bosses is a vented space allowing exhaled gas to escape when exhale valve 'flap' 341 is deflected away from valve seat 343. Voice coil drive connections are shown at the top right of this 'exhalation control valve' 340 and would be connected to a connector such as is indicated attached to the side of mid plate 306 in Figures 3A and 3B.

Advantages of this design over the aforementioned solenoid armature 347 with its actuator rod 346 include its simpler construction and maintenance. Another is the inherently sprung exhale valve 'flap' 341 support; with accurate setting of mounting boss height, that spring force can provide the required default exhalation pressure. Gravity is not needed to hold the valve shut so any orientation works well. A spring pre-tension could be applied for those purposes on the solenoid valve implementation but would add to design complexity.

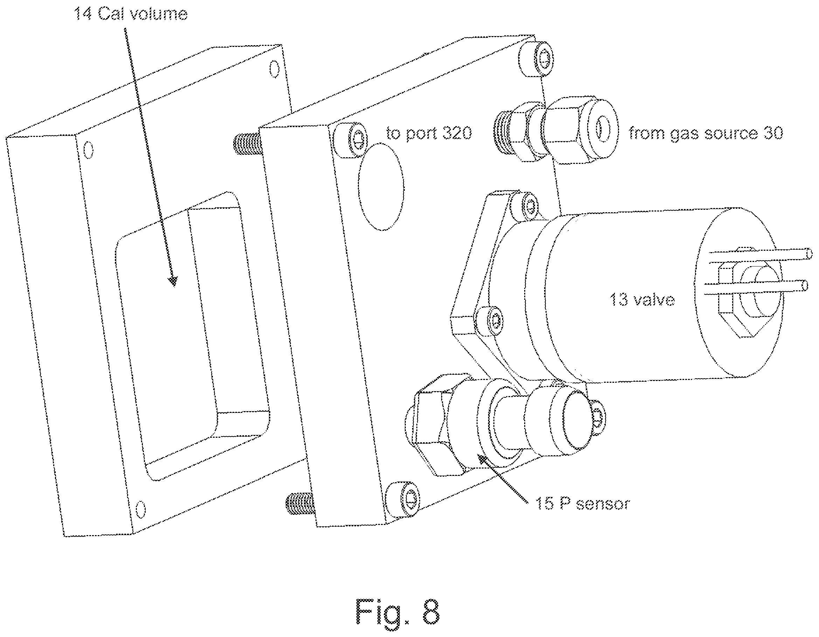

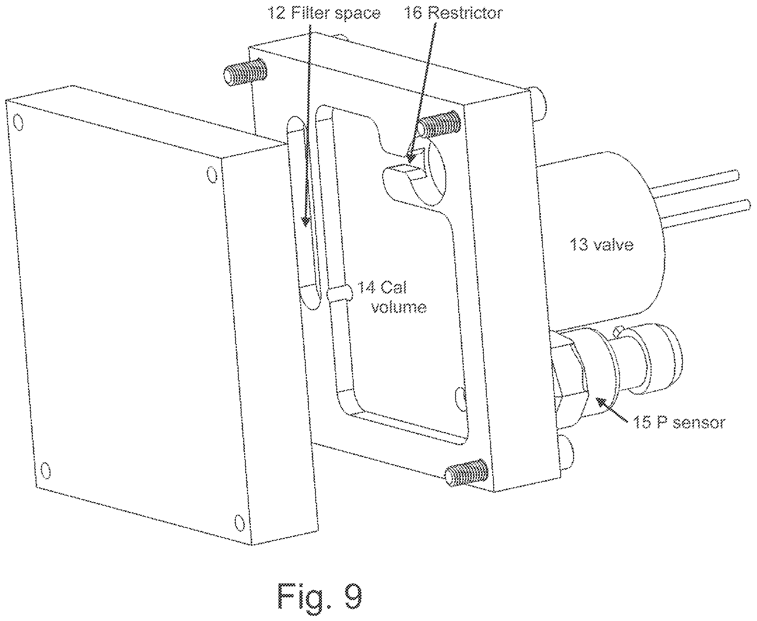

Figures 8 and 9 show exploded views of a representative embodiment of GFC module. Calibration volume 14 is hollowed out of both top and bottom slabs to yield sufficient volume for averaging pressure fluctuations at high flow rates. A single on/off valve 13 is shown here although as described in the context of Figure 1 where a wide flow range must be handled, more than one valve may be placed in parallel to enable both high valve speed and low valve speed regimes to be handled with high accuracy, or a proportional valve may be used. The GFC calibrated volume 14 may also be formed from tubular shapes or conduits using standard plumbing fittings if desired.

A filter 12 may be placed in the line from gas source 30, or alternatively filter media such as metal turnings or metal mesh or cloth may be packed into the space shown between gas source 30 fitting and the input to solenoid valve 13 in Figure 9.

The foregoing description and accompanying drawings set forth a number of representative embodiments at the present time. Various modifications, additions and alternative designs will, of course, become apparent to those skilled in the art in light of the foregoing teachings without departing from the scope of the appended claims.

## Claims

1. A system to supply breathing gas to patient airways, comprising:
a gas distribution manifold (310) comprising at least one patient interface port (380);
at least one controllable source of a breathing gas comprising a pressurized tank (30) of the breathing gas;
a gas flow controller (10) in fluid connection between the pressurized tank and the gas distribution manifold, the gas flow controller volumetrically measuring and controlling in real time flow of the breathing gas, the gas flow controller comprising a calibration volume (14), a temperature sensor (18) in operative connection with the calibration volume, a pressure sensor (15) in fluid connection with the calibration volume, a controllable valve (13) in fluid connection between the source of the breathing gas and the calibration volume, and a restrictor (16) downstream from the calibration volume and in fluid connection between the calibration volume and the gas distribution manifold; and
a control system (510) in operative connection with the gas flow controller and the gas distribution manifold,
**characterized in that** the control system is configured to control the controllable valve to vary the gas flow rate of breathing gas from the source of breathing gas in an oscillatory manner therethrough such that an average gas pressure in the calibration volume correlates with an average level of gas flow, and wherein variations in calibration volume pressure induced by varying the gas flow rate of the breathing gas through the controllable valve in the determined manner are used by the control system to determine and control an average molecular rate of gas flow to the at least one patient interface port in real time.

2. The system of claim 1 wherein the gas distribution manifold comprises an interstitial space (352) fluidly connected to the at least one patient interface port which is configured to be placed in fluid connection with a patient breathing tube, at least a first pressure sensor (350) in fluid connection with the interstitial space, an exhalation valve (340) in fluid connection with the interstitial space, a controllable proportioning exhalation valve drive operatively connected to the exhalation valve to dynamically control pressures within the interstitial space, wherein the exhalation valve drive allows opening of the exhalation valve upon patient exhalation in case of system or power failure, an ambient air inlet port (315) in fluid connection with the interstitial space and comprising a one-way valve (316) to enable patient inhalation in case of system or power failure, and a gas inlet port (320) in fluid connection with the interstitial space via a one-way valve (321); wherein the gas flow controller is in fluid connection between the source of breathing gas and the gas inlet port, and wherein the control system is in operative connection with the exhalation valve drive and in operative connection with the at least a first pressure sensor.

3. The system of claim 2 wherein the control system is configured to vary the gas flow rate of breathing gas from the source of breathing gas to a patient via the at least one patient interface port over time.

4. The system of claim 2 wherein the controllable valve is the only valve controlled via the control system between the outlet of the pressurized tank and the gas inlet port to volumetrically measure and control the average molecular rate of the breathing gas flow to the at least one patient interface port which permits the molecular rate of breathing gas flow to the at least one patient interface port to vary within process-compatible limits around the average molecular rate of the breathing gas flow in an oscillatory manner.

5. The system of claim 1 wherein the calibration volume and the restrictor are formed via a sealed connection of a plurality of gas flow controller blocks, at least one of the gas flow controller blocks comprising a port for connection of the controllable valve and at least one of the gas flow controller blocks comprising a port for the gas flow controller pressure sensor.

6. The system of claim 5 wherein the calibration volume is formed from cylindrical pipe, and the restrictor comprises at least one fitting and tubing connecting the cylindrical pipe to the gas inlet port of the gas distribution manifold.

7. The system of any one of claim 1 to 6 further comprising at least a second controllable source of a breathing gas in fluid connection with the gas inlet port, the at least a second controllable source of the breathing gas comprising a fan (110), a bellows or a piston in operative connection with the control system, optionally wherein the at least a second controllable source of the breathing gas comprises a fan in operative connection with the control system and the gas distribution manifold is formed via sealed connection of a plurality of manifold blocks.

8. The system of any one of claims 1 to 7 wherein the gas distribution manifold is formed via sealed connection of a plurality of manifold blocks, optionally wherein the interstitial space is formed by the sealed connection of at least two of the plurality of manifold blocks.

9. The system of any one of claims 2 to 8 wherein the interstitial space comprises an inhalation interstitial space and an exhalation interstitial space.

10. The system of claim 9 wherein the at least one patient interface port is an inhalation patient interface port and is in fluid connection with the inhalation interstitial space, the gas inlet port and the ambient air inlet port also being in fluid communication with the inhalation interstitial space, and the system further comprises an exhalation patient port which is configured to be connected to an exhalation breathing tube and is in fluid connection with the exhalation interstitial space, an exhalation valve port being in fluid connection with the exhalation interstitial space to which the exhalation valve is connected.

11. The system of claim 10 wherein at least one pressure sensor port is in fluid connection with one of the inhalation patient port or with the exhalation interstitial space.

12. The system of any one of claims 2 to 11 further comprising an overpressure valve which releases overpressure in fluid connection with the interstitial space.

13. The system of claim 9 wherein an overpressure valve is in fluid connection with the exhalation interstitial space.

## Patentansprüche

1. System zur Versorgung von Patientenatemwegen mit Atemgas, umfassend:
einen Gasverteilerkrümmer (310), umfassend wenigstens einen Patientenschnittstellenanschluss (380);
wenigstens eine steuerbare Quelle eines Atemgases, umfassend einen Drucktank (30) des Atemgases;
einen Gasflussregler (10) in Fluidverbindung zwischen dem Drucktank und dem Gasverteilerkrümmer, wobei der Gasflussregler den Fluss des Atemgases volumetrisch misst und in Echtzeit steuert, wobei der Gasflussregler ein Kalibriervolumen (14), einen Temperatursensor (18) in betriebsmäßiger Verbindung mit dem Kalibriervolumen, einen Drucksensor (15) in Fluidverbindung mit dem Kalibriervolumen, ein steuerbares Ventil (13) in Fluidverbindung zwischen der Atemgasquelle und dem Kalibriervolumen und einen Restriktor (16) nachgeschaltet dem Kalibriervolumen und in Fluidverbindung zwischen dem Kalibriervolumen und dem Gasverteilerkrümmer umfasst; und
ein Steuersystem (510) in betriebsmäßiger Verbindung mit dem Gasflussregler und dem Gasverteilerkrümmer,
**dadurch gekennzeichnet, dass** das Steuersystem konfiguriert ist, um das steuerbare Ventil zu steuern, um die Gasflussrate des Atemgases von der Atemgasquelle oszillierend durch dieses zu variieren, so dass ein durchschnittlicher Gasdruck im Kalibriervolumen mit einem durchschnittlichen Gasflusspegel korreliert, und wobei Druckschwankungen im Kalibriervolumen, die durch Variieren der Gasflussrate des Atemgases durch das steuerbare Ventil auf die bestimmte Weise induziert werden, vom Steuersystem verwendet werden, um eine durchschnittliche Molekularrate des Gasflusses zum wenigstens einen Patientenschnittstellenanschluss in Echtzeit zu bestimmen und zu steuern.

2. System nach Anspruch 1, wobei der Gasverteilerkrümmer einen Zwischenraum (352) umfasst, der fluidisch mit dem wenigstens einen Patientenschnittstellenanschluss verbunden ist, der konfiguriert ist, um in Fluidverbindung mit einem Patientenatemrohr gebracht zu werden, wenigstens einen ersten Drucksensor (350) in Fluidverbindung mit dem Zwischenraum, ein Ausatemventil (340) in Fluidverbindung mit dem Zwischenraum, einen steuerbaren proportionierenden Ausatemventilantrieb, der betriebsmäßig mit dem Ausatemventil verbunden ist, um Drücke innerhalb des Zwischenraums dynamisch zu steuern, wobei der Ausatemventilantrieb das Öffnen des Ausatemventils bei Patientenexhalation im Falle eines System- oder Stromausfalls ermöglicht, einen Umgebungslufteinlassanschluss (315) in Fluidverbindung mit dem Zwischenraum und umfassend ein Einwegventil (316), um die Patienteninhalation im Falle eines System- oder Stromausfalls zu ermöglichen, und einen Gaseinlassanschluss (320) in Fluidverbindung mit dem Zwischenraum über ein Einwegventil (321);
wobei der Gasflussregler in Fluidverbindung zwischen der Atemgasquelle und dem Gaseinlassanschluss ist, und wobei das Steuersystem in betriebsmäßiger Verbindung mit dem Ausatemventilantrieb und in betriebsmäßiger Verbindung mit dem wenigstens ersten Drucksensor ist.

3. System nach Anspruch 2, wobei das Steuersystem konfiguriert ist, um die Gasflussrate des Atemgases von der Atemgasquelle zu einem Patienten über den wenigstens einen Patientenschnittstellenanschluss über die Zeit zu variieren.

4. System nach Anspruch 2, wobei das steuerbare Ventil das einzige Ventil ist, das über das Steuersystem zwischen dem Auslass des Drucktanks und dem Gaseinlassanschluss gesteuert wird, um die durchschnittliche Molekularrate des Atemgasflusses zu dem wenigstens einen Patientenschnittstellenanschluss volumetrisch zu messen und zu steuern, was es ermöglicht, dass die Molekularrate des Atemgasflusses zu dem wenigstens einen Patientenschnittstellenanschluss innerhalb prozesskompatibler Grenzen um die durchschnittliche Molekularrate des Atemgasflusses oszillierend variiert.

5. System nach Anspruch 1, wobei das Kalibriervolumen und der Restriktor durch eine abgedichtete Verbindung einer Vielzahl von Gasflussreglerblöcken gebildet werden, wobei wenigstens einer der Gasflussreglerblöcke einen Anschluss zur Verbindung des steuerbaren Ventils umfasst und wenigstens einer der Gasflussreglerblöcke einen Anschluss für den Gasflussregler-Drucksensor umfasst.

6. System nach Anspruch 5, wobei das Kalibriervolumen aus einem zylindrischen Rohr gebildet wird, und der Restriktor wenigstens eine Armatur und eine Verrohrung umfasst, die das zylindrische Rohr mit dem Gaseinlassanschluss des Gasverteilerkrümmers verbinden.

7. System nach einem der Ansprüche 1 bis 6, ferner umfassend wenigstens eine zweite steuerbare Quelle eines Atemgases in Fluidverbindung mit dem Gaseinlassanschluss, wobei die wenigstens zweite steuerbare Quelle des Atemgases einen Ventilator (110), einen Balg oder einen Kolben in betriebsmäßiger Verbindung mit dem Steuersystem umfasst, wobei optional die wenigstens zweite steuerbare Quelle des Atemgases einen Ventilator in betriebsmäßiger Verbindung mit dem Steuersystem umfasst und der Gasverteilerkrümmer durch abgedichtete Verbindung einer Vielzahl von Krümmerblöcken gebildet wird.

8. System nach einem der Ansprüche 1 bis 7, wobei der Gasverteilerkrümmer durch abgedichtete Verbindung einer Vielzahl von Krümmerblöcken gebildet wird, wobei optional der Zwischenraum durch die abgedichtete Verbindung von wenigstens zwei der Vielzahl von Krümmerblöcken gebildet wird.

9. System nach einem der Ansprüche 2 bis 8, wobei der Zwischenraum einen Inhalation-Zwischenraum und einen Ausatem-Zwischenraum umfasst.

10. System nach Anspruch 9, wobei der wenigstens eine Patientenschnittstellenanschluss ein Inhalation-Patientenschnittstellenanschluss ist und in Fluidverbindung mit dem Inhalation-Zwischenraum ist, wobei der Gaseinlassanschluss und der Umgebungslufteinlassanschluss ebenfalls in Fluidkommunikation mit dem Inhalation-Zwischenraum sind, und das System ferner einen Ausatem-Patientenanschluss umfasst, der konfiguriert ist, um mit einem Ausatem-Atemrohr verbunden zu werden und in Fluidverbindung mit dem Ausatem-Zwischenraum ist, wobei ein Ausatemventilanschluss in Fluidverbindung mit dem Ausatem-Zwischenraum ist, mit dem das Ausatemventil verbunden ist.

11. System nach Anspruch 10, wobei wenigstens ein Drucksensoranschluss in Fluidverbindung mit einem der Inhalation-Patientenanschlüsse oder mit dem Ausatem-Zwischenraum ist.

12. System nach einem der Ansprüche 2 bis 11, ferner umfassend ein Überdruckventil, das Überdruck in Fluidverbindung mit dem Zwischenraum ablässt.

13. System nach Anspruch 9, wobei ein Überdruckventil in Fluidverbindung mit dem Ausatem-Zwischenraum ist.

## Revendications

1. Système d'alimentation en gaz respiratoire des voies respiratoires d'un patient, comprenant :
un collecteur de distribution de gaz (310) comprenant au moins un orifice d'interface patient (380) ;
au moins une source commandable d'un gaz respiratoire comprenant un réservoir sous pression (30) du gaz respiratoire ;
un régulateur de débit de gaz (10) en connexion fluidique entre le réservoir sous pression et le collecteur de distribution de gaz, le régulateur de débit de gaz mesurant et commandant volumétriquement en temps réel le débit du gaz respiratoire, le régulateur de débit de gaz comprenant un volume d'étalonnage (14), un capteur de température (18) en connexion fonctionnelle avec le volume d'étalonnage, un capteur de pression (15) en connexion fluidique avec le volume d'étalonnage, une vanne commandable (13) en connexion fluidique entre la source du gaz respiratoire et le volume d'étalonnage, et un restricteur (16) en aval du volume d'étalonnage et en connexion fluidique entre le volume d'étalonnage et le collecteur de distribution de gaz ; et
un système de commande (510) en liaison fonctionnelle avec le régulateur de débit de gaz et le collecteur de distribution de gaz,
**caractérisé en ce que** le système de commande est configuré pour commander la vanne commandable afin de faire varier le débit de gaz du gaz respiratoire provenant de la source de gaz respiratoire de manière oscillatoire à travers celle-ci de telle sorte qu'une pression de gaz moyenne dans le volume d'étalonnage soit corrélée à un niveau moyen de débit de gaz, et les variations de la pression du volume d'étalonnage induites par la variation du débit de gaz du gaz respiratoire à travers la vanne commandable de la manière déterminée étant utilisées par le système de commande pour déterminer et commander un taux moléculaire moyen de débit de gaz vers l'au moins un orifice d'interface patient en temps réel.

2. Système selon la revendication 1, dans lequel le collecteur de distribution de gaz comprend un espace interstitiel (352) connecté fluidiquement à l'au moins un orifice d'interface patient qui est configuré pour être placé en connexion fluidique avec un tube respiratoire d'un patient, au moins un premier capteur de pression (350) en connexion fluidique avec l'espace interstitiel, une valve d'expiration (340) en connexion fluidique avec l'espace interstitiel, une commande de valve d'expiration à dosage réglable connectée fonctionnellement à la valve d'expiration pour commander dynamiquement les pressions dans l'espace interstitiel, la commande de valve d'expiration permettant l'ouverture de la valve d'expiration lors de l'expiration du patient en cas de panne du système ou de courant, un orifice d'entrée d'air ambiant en connexion fluidique avec l'espace interstitiel et comprenant une valve unidirectionnelle pour permettre l'inhalation du patient en cas de panne du système ou de courant, et un orifice d'entrée de gaz en connexion fluidique avec l'espace interstitiel via une valve unidirectionnelle (321) ;
dans lequel le régulateur de débit de gaz est en connexion fluidique entre la source de gaz respiratoire et l'orifice d'entrée de gaz, et dans lequel le système de commande est en connexion fonctionnelle avec la commande de la valve d'expiration et en connexion fonctionnelle avec l'au moins un premier capteur de pression.

3. Système selon la revendication 2, dans lequel le système de commande est configuré pour faire varier le débit de gaz respiratoire depuis la source de gaz respiratoire vers un patient via l'au moins un orifice d'interface patient au fil du temps.

4. Système selon la revendication 2 dans lequel la vanne commandable est la seule vanne commandée par le système de commande entre la sortie du réservoir sous pression et l'orifice d'entrée de gaz pour mesurer et commander volumétriquement le débit moléculaire moyen du flux de gaz respiratoire vers l'au moins un orifice d'interface patient, ce qui permet au débit moléculaire du flux de gaz respiratoire vers l'au moins un orifice d'interface patient de varier dans des limites compatibles avec le processus autour du débit moléculaire moyen du flux de gaz respiratoire de manière oscillatoire.

5. Système selon la revendication 1 dans lequel le volume d'étalonnage et le restricteur sont formés via une connexion étanche d'une pluralité de blocs de régulateur de débit de gaz, au moins l'un des blocs de régulateur de débit de gaz comprenant un orifice pour la connexion de la vanne commandable et au moins l'un des blocs de régulateur de débit de gaz comprenant un orifice pour le capteur de pression du dispositif de commande de débit de gaz.

6. Système selon la revendication 5 dans lequel le volume d'étalonnage est formé à partir d'un tuyau cylindrique, et le restricteur comprend au moins un raccord et un tube reliant le tuyau cylindrique à l'orifice d'entrée de gaz du collecteur de distribution de gaz.

7. Système selon l'une quelconque des revendications 1 à 6 comprenant également au moins une seconde source contrôlable d'un gaz respiratoire en connexion fluidique avec l'orifice d'entrée de gaz, l'au moins une seconde source contrôlable du gaz respiratoire comprenant un ventilateur (110), un soufflet ou un piston en connexion fonctionnelle avec le système de commande, éventuellement dans lequel l'au moins une seconde source contrôlable du gaz respiratoire comprend un ventilateur en connexion fonctionnelle avec le système de commande et le collecteur de distribution de gaz est formé par l'intermédiaire d'une connexion étanche d'une pluralité de blocs collecteurs.

8. Système selon l'une quelconque des revendications 1 à 7 dans lequel le collecteur de distribution de gaz est formé par l'intermédiaire d'une connexion étanche d'une pluralité de blocs collecteurs, éventuellement dans lequel l'espace interstitiel est formé par la connexion étanche d'au moins deux de la pluralité de blocs collecteurs.

9. Système selon l'une quelconque des revendications 2 à 8, dans lequel l'espace interstitiel comprend un espace interstitiel d'inhalation et un espace interstitiel d'expiration.

10. Système selon la revendication 9 dans lequel l'au moins un orifice d'interface patient est un orifice d'interface patient d'inhalation et est en communication fluidique avec l'espace interstitiel d'inhalation, l'orifice d'entrée de gaz et l'orifice d'entrée d'air ambiant étant également en communication fluidique avec l'espace interstitiel d'inhalation, et le système comprend également un orifice patient d'expiration qui est configuré pour être connecté à un tube respiratoire d'expiration et est en connexion fluidique avec l'espace interstitiel d'expiration, un orifice de valve d'expiration étant en connexion fluidique avec l'espace interstitiel d'expiration auquel la valve d'expiration est connectée.

11. Système selon la revendication 10 dans lequel au moins un orifice de capteur de pression est en connexion fluidique avec l'un des orifices patient d'inhalation ou avec l'espace interstitiel d'expiration.

12. Système selon l'une quelconque des revendications 2 à 11 comprenant également une soupape de surpression qui libère la surpression en connexion fluidique avec l'espace interstitiel.

13. Système selon la revendication 9 dans lequel une soupape de surpression est en connexion fluidique avec l'espace interstitiel d'expiration.
